(19) Europäisches Patentamt
European Patent Office
Office européen des brevets

(11) EP 2 103 615 A1

(12) EUROPÄISCHE PATENTANMELDUNG

(43) Veröffentlichungstag:
**23.09.2009 Patentblatt 2009/39**

(51) Int Cl.:
***C07D 491/113*** *(2006.01)* ***A01N 43/90*** *(2006.01)*

(21) Anmeldenummer: **08153002.4**

(22) Anmeldetag: **19.03.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Bayer CropScience AG**
**40789 Monheim (DE)**

(72) Erfinder: **Die Erfindernennung liegt noch nicht vor**

(54) **4'4'-Dioxaspiro-spirocyclisch substituierte Tetramate**

(57) Die vorliegende Erfindung betrifft neue 4'4'-Dioxaspiro-spirocyclisch substituierte Tetramate, der Formel (I)

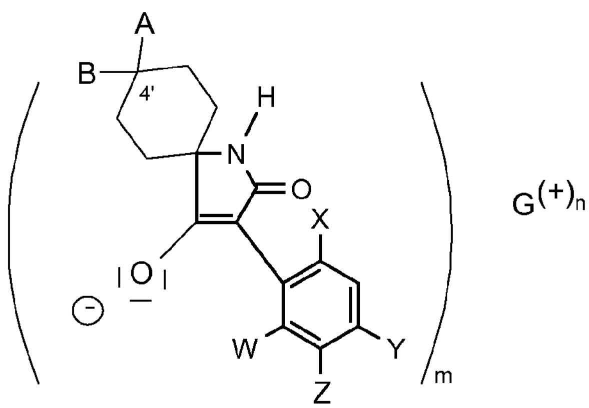

(I)

in welcher A, B, G, m, n, W, X, Y und Z
mehrere Verfahren zu ihrer Herstellung und ihre Verwendung als Schädlingsbekämpfungsmittel und/oder Herbizide. Auch Gegenstand der Erfindung sind selektiv herbizide Mittel, die 4'4'-Dioxaspiro-spirocyclisch substituierte Tetramate einerseits und eine die Kulturpflanzenverträglichkeit verbessernde Verbindung andererseits enthalten.

Die vorliegende Erfindung betrifft auch neue wasserlösliche Konzentrate von 4'4',Dioxaspiro-spirocyclisch substituierten Tetramaten und deren Enolen, Verfahren zur Herstellung dieser Formulierungen und ihre Verwendung als Schädlingsbekämpfungsmittel und / oder Herbizide.

Die vorliegende Erfindung betrifft weiterhin die Steigerung der Wirkung von Pflanzenschutzmitteln enthaltend insbesondere 4'4'-Dioxaspiro-spirocyclisch substituierte Tetramate, durch die Zugabe von Ammonium- oder Phosphoniumsalzcn und gcgcbcncfalls Penetrationsförderem, die entsprechenden Mittel, Verfahren zur ihrer Herstellung und ihre Anwendung im Pflanzenschutz als Insektizide und/oder Akarizide und/oder zur Verhinderung von unerwünschten Pflanzenwuchs.

EP 2 103 615 A1

## Beschreibung

Die vorliegende Erfindung betrifft neue 4'4'-Dioxaspiro-spirocyclisch substituierte Tetramate, mehrere Verfahren zu ihrer Herstellung und ihre Verwendung als Schädlingsbekämpfungsmittel und/oder Herbizide. Auch Gegenstand der Erfindung sind selektiv herbizide Mittel, die 4'4'-Dioxaspiro-spirocyclisch substituierte Tetramate einerseits und eine die Kulturpflanzenverträglichkeit verbessernde Verbindung andererseits enthalten.

Die vorliegende Erfindung betrifft auch neue wasserlösliche Konzentrate (SL-Formulierungen) von 4'4'-Dioxaspiro-spirocyclisch substituierten Tetramaten und deren Enolen, Verfahren zur Herstellung dieser Formulierungen und ihre Verwendung als Schädlingsbekämpfungsmittel und / oder Herbizide.

Die vorliegende Erfindung betrifft weiterhin die Steigerung der Wirkung von Pflanzenschutzmitteln enthaltend insbesondere 4'4'-Dioxaspiro-spirocyclisch substituierte Tetramate, durch die Zugabe von Ammonium- oder Phosphoniumsalzen und gegebenefalls Penetrationsförderern, die entsprechenden Mittel, Verfahren zur ihrer Herstellung und ihre Anwendung im Pflanzenschutz als Insektizide und/oder Akarizide und/oder zur Verhinderung von unerwünschten Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1 H-Arylpyrrolidin-dion-Derivate (EP-A-456 063, EP-A-521 334, EP-A-596 298, EP-A-613 884, EP-A-613 885, WO 94/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869 und WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049596, WO 05/066125, WO 05/092897, WO 06/000355, WO 06/029799, WO 06/056281, WO 06/056282, WO 06/089633, WO 07/048545, WO 07/073856, WO 07/096058, WO 07/121868 und WO 07/140881. Außerdem sind ketalsubstituierte 1-H-Arylpyrrolidin-2,4-dione aus WO 99/16748 und (spiro)-ketalsubstituierte N-Alkoxy-alkoxysubstituierte Aryl-pyrrolidindione aus JP-A-14 205 984 und Ito M. et. al Bioscience, Biotechnology and Biochemistry 67, 1230-1238, (2003) bekannt. Der Zusatz von Safenern zu Ketoenolen ist ebenfalls prinzipiell aus der WO 03/013249 bekannt. Außerdem sind aus WO 06/024411 herbizide Mittel enthaltend Ketoenole bekannt.

Die herbizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (I)

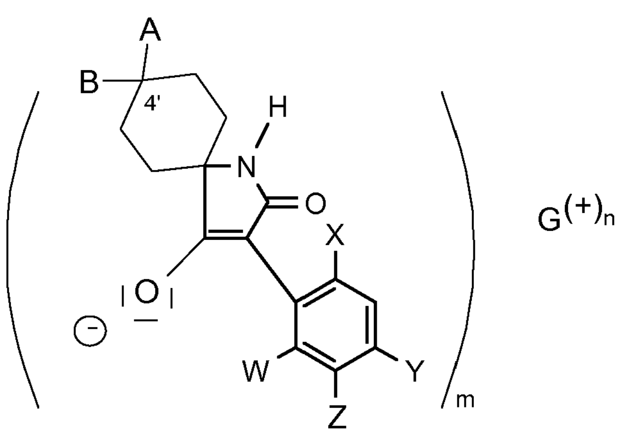

(I)

gefunden,
in welcher

W für Wasserstoff, Alkyl, Alkenyl, Alkinyl, Halogen, Alkoxy, Halogenalkyl, Halogenalkoxy oder Cyano steht,

X für Halogen, Alkyl, Alkenyl, Alkinyl, Alkoxy, Halogenalkyl, Halogenalkoxy, Nitro oder Cyano steht,

Y und Z unabhängig voneinander für Wasserstoff, Alkyl, Alkenyl, Alkinyl, Alkoxy, Halogen, Halogenalkyl, Halogenalkoxy, Cyano oder Nitro steht,

A und B und das Kohlenstoffatom, an das sie gebunden sind, für jeweils gegebenenfalls durch Alkyl, Halogenalkyl, Alkoxy, Alkoxyalkyl oder gegebenenfalls substituiertes Phenyl substituiertes fünf- bis siebengliedriges Ketal steht,

G für ein Metallionenäquivalent oder Ammoniumion steht,

m für die Zahl 1 oder 2 steht,

n für die Zahl 1 oder 2 steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Tautomeren- und Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren und tautomeren Verbindungen gemeint sind.

Weiterhin wurde gefunden, dass man die neuen Verbindungen der Formel (I) nach einem der im folgenden beschriebenen Verfahren erhält:

(A) Man erhält substituierte 4'4'-Dioxaspiro-spirocyclisch substituierte Tetramate der Formel (I)

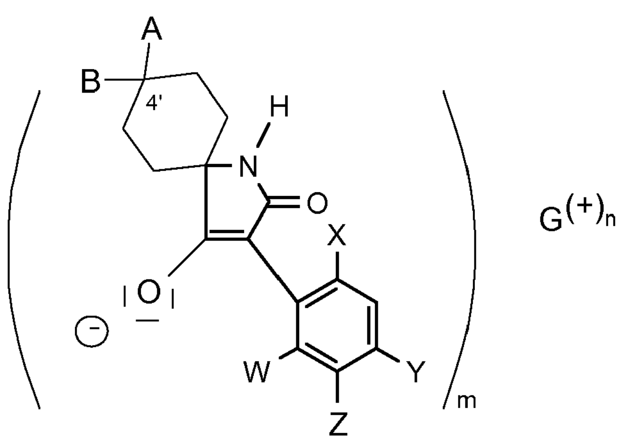

(I)

in welcher
A, B, D, G, m, n, W, X, Y und Z die oben angegebenen Bedeutungen haben,
wenn man
N-Acylaminosäureester der Formel (II)

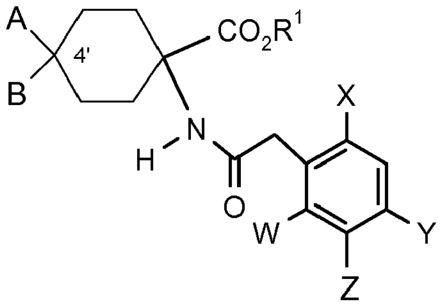

(II)

in welcher

A, B, W, X, Y und Z die oben angegebenen Bedeutungen haben,

und

$R^1$ für Alkyl (bevorzugt $C_1$-$C_6$-Alkyl) steht,

in Gegenwart eines Verdünnungsmittels und in Gegenwart einer Metallbase intramolekular kondensiert.

(B) Weiterhin wurde gefunden, dass man Verbindungen der oben gezeigten Formel (I), in welcher A, B, G, m, n, W, X, Y und Z die oben angegebenen Bedeutungen haben, erhält, wenn man Verbindungen der Formel (I'),

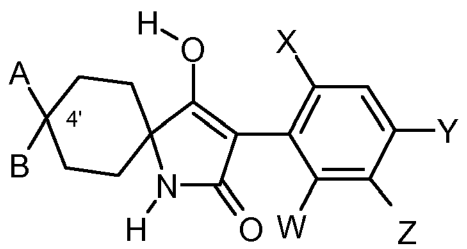

(I')

in welchen A, B, W, X, Y und Z die oben angegebenen Bedeutungen haben, jeweils

α) mit Metallverbindungen der Formeln (III) oder (IV)

$G(OR^2)_n$ (III) , $G(H)_n$ (IV)

in welchen

G für ein ein- oder zweiwertiges Metall (bevorzugt ein Alkali- oder Erdalkalimetall wie Lithium, Natrium, Kalium, Cäsium, Magnesium oder Calcium),

n für die Zahl 1 oder 2 und

$R^2$ für Wasserstoff oder Alkyl (bevorzugt $C_1$-$C_8$-Alkyl) steht,

gegebenenfalls in Gegenwart eines Verdünnungsmittels umsetzt
oder

β) mit Aminen der Formel (V) oder Ammoniumverbindungen der Formel (VI)

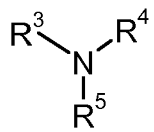 (V) , 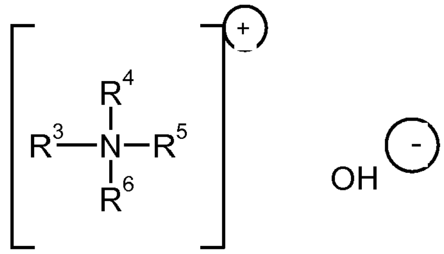 (VI)

in welchen
$R^3$, $R^4$, $R^5$, $R^6$ unabhängig voneinander für Wasserstoff, $C_1$-$C_8$ Alkyl, $C_1$-$C_4$-Alkoxy-$C_2$-$C_4$-alkyl, Poly-($C_1$-$C_4$-Alkoxy)-$C_2$-$C_4$-alkyl, $C_3$-$C_8$-Alkenyl, $C_1$-$C_8$-Alkoxy oder gegebenenfalls durch Halogen, Alkyl oder Alkoxy substituiertes Benzyl steht,
gegebenenfalls in Gegenwart eines Verdünnungsmittels umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten

(a') mindestens ein 4'4'-Dioxaspiro-spirocyclisch substituiertes Tetramat der Formel (I), in welcher A, B, G, m, n, W, X, Y und Z die oben angegebene Bedeutung haben
und

(b') zumindest eine die Kulturpflanzen-Verträglichkeit verbessernde Verbindung aus der folgenden Gruppe von Verbindungen:

4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methylhexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenylethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methylphenyl)-harnstoff (Daimuron, Dymron), 3,6-Dichlor-2-methoxy-benzoesäure (Dicamba), Piperidin-1-thiocarbonsäure-S-1-methyl-1-phenylethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlor-phenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-a-trifluor-acetophenonoxim (Fluxofenim), 3-Dichloracetyl-5-(2-furanyl)-2,2-dimethyloxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlorphenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlorbenzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-ylester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxoprop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,

und/oder eine der folgenden durch allgemeine Formeln definierten Verbindungen der allgemeinen Formel (IIa)

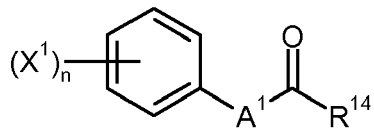

(IIa)

oder der allgemeinen Formel (IIb)

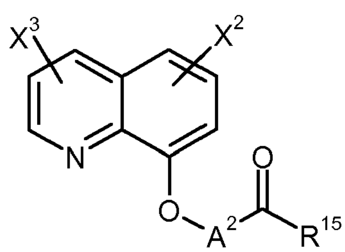

(IIb)

oder der Formel (IIc)

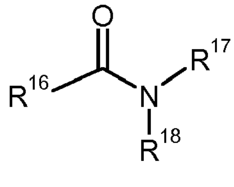

(IIc)

wobei

n für eine Zahl zwischen 0 und 5 steht,

$A^1$ für eine der nachstehend skizzierten divalenten heterocyclischen Gruppierungen steht,

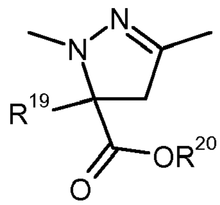 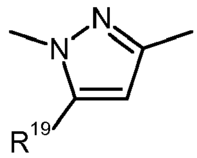 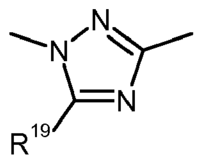 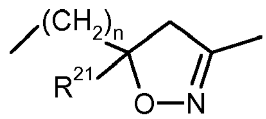

n für eine Zahl zwischen 0 und 5 steht,

$A^2$ für gegebenenfalls durch $C_1$-$C_4$-Alkyl und/oder $C_1$-$C_4$-Alkoxy- carbonyl substituiertes Alkandiyl mit 1 oder 2 Kohlenstoffatomen steht,

$R^{14}$ für Hydroxy, Mercapto, Amino, $C_1$-$C_6$-Alkoxy, $C_1$-$C_6$-Alkylthio, $C_1$-$C_6$-Alkylamino oder Di-($C_1$-$C_4$-alkyl)-amino steht,

$R^{15}$ für Hydroxy, Mercapto, Amino, $C_1$-$C_6$-Alkoxy, $C_1$-$C_6$-Alkylthio, $C_1$-$C_6$-Alkylamino oder Di-($C_1$-$C_4$-alkyl)-amino steht,

$R^{16}$ für jeweils gegebenenfalls durch Fluor, Chlor und/oder Brom substituiertes $C_1$-$C_4$-Alkyl steht,

$R^{17}$ für Wasserstoff jeweils gegebenenfalls durch Fluor, Chlor und/oder Brom substituiertes $C_1$-$C_6$-Alkyl, $C_2$-$C_6$-Alkenyl oder $C_2$-$C_6$-Alkinyl, $C_1$-$C_4$-Alkoxy-$C_1$-$C_4$-alkyl, Dioxolanyl-$C_1$-$C_4$-alkyl, Furyl, Furyl-$C_1$-$C_4$-alkyl, Thienyl, Thiazolyl, Piperidinyl, oder gegebenenfalls durch Fluor, Chlor und/oder Brom oder $C_1$-$C_4$-Alkyl substituiertes Phenyl steht,

$R^{18}$ für Wasserstoff jeweils gegebenenfalls durch Fluor, Chlor und/oder Brom substituiertes $C_1$-$C_6$-Alkyl, $C_2$-$C_6$-Alkenyl oder $C_2$-$C_6$-Alkinyl, $C_1$-$C_4$-Alkoxy-$C_1$-$C_4$-alkyl, Dioxolanyl-$C_1$-$C_4$-alkyl, Furyl, Furyl-$C_1$-$C_4$-alkyl, Thienyl, Thiazolyl, Piperidinyl, oder gegebenenfalls durch Fluor, Chlor und/oder Brom oder $C_1$-$C_4$-Alkyl substituiertes Phenyl, oder zusammen mit $R^{17}$ für jeweils gege- benenfalls durch $C_1$-$C_4$-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes $C_3$-$C_6$-Alkandiyl oder $C_2$-$C_5$-Oxaalkandiyl steht,

$R^{19}$ für Wasserstoff, Cyano, Halogen, oder für jeweils gegebenenfalls durch Fluor, Chlor und/oder Brom substituiertes $C_1$-$C_4$-Alkyl, $C_3$-$C_6$-Cycloalkyl oder Phenyl steht,

$R^{20}$ für Wasserstoff, gegebenenfalls durch Hydroxy, Cyano, Halogen oder $C_1$-$C_4$-Alkoxy substituiertes $C_1$-$C_6$-Alkyl, $C_3$-$C_6$-Cycloalkyl oder Tri-($C_1$-$C_4$-alkyl)-silyl steht,

$R^{21}$ für Wasserstoff, Cyano, Halogen, oder für jeweils gegebenenfalls durch Fluor, Chlor und/oder Brom substituiertes $C_1$-$C_4$-Alkyl, $C_3$-$C_6$-Cycloalkyl oder Phenyl steht,

$X^1$ für Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy steht,

$X^2$ für Wasserstoff, Cyano, Nitro, Halogen, $C_1$-$C_4$-Alkyl, $C_1$-$C_4$-Halogenalkyl, $C_1$-$C_4$-Alkoxy oder $C_1$-$C_4$-Halogenalkoxy steht,

$X^3$ für Wasserstoff, Cyano, Nitro, Halogen, $C_1$-$C_4$-Alkyl, $C_1$-$C_4$-Halogenalkyl, $C_1$-$C_4$-Alkoxy oder $C_1$-$C_4$-Halogenalkoxy steht,

und/oder die folgenden durch allgemeine Formeln definierten Verbindungen
der allgemeinen Formel (IId)

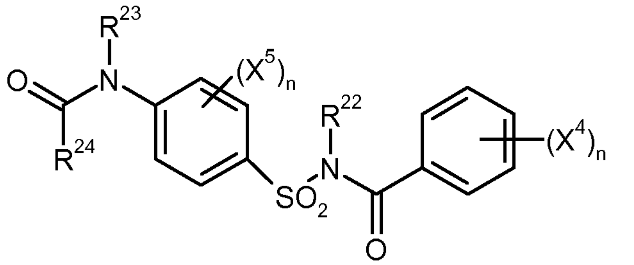

(IId)

oder der allgemeinen Formel (IIe)

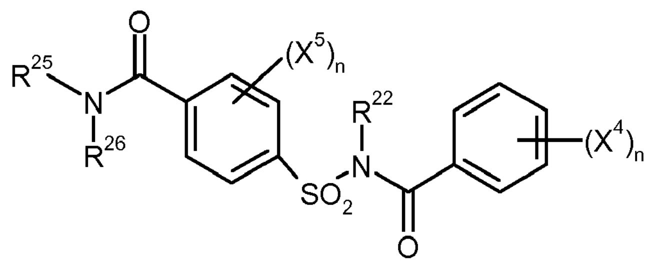

(IIe)

wobei

n für eine Zahl zwischen 0 und 5 steht,

$R^{22}$ für Wasserstoff oder $C_1$-$C_4$-Alkyl steht,

$R^{23}$ für Wasserstoff oder $C_1$-$C_4$-Alkyl steht,

$R^{24}$ für Wasserstoff, jeweils gegebenenfalls durch Cyano, Halogen oder $C_1$-$C_4$-Alkoxy substituiertes $C_1$-$C_6$-Alkyl, $C_1$-$C_6$-Alkoxy, $C_1$-$C_6$-Alkylthio, $C_1$-$C_6$-Alkylamino oder Di-($C_1$-$C_4$-alkyl)-amino, oder jeweils gegebenenfalls durch Cyano, Halogen oder $C_1$-$C_4$-Alkyl substituiertes $C_3$-$C_6$-Cyclo-alkyl, $C_3$-$C_6$-Cycloalkyloxy, $C_3$-$C_6$-Cycloalkylthio oder $C_3$-$C_6$-Cycloalkylamino steht,

$R^{25}$ für Wasserstoff, gegebenenfalls durch Cyano, Hydroxy, Halogen oder $C_1$-$C_4$-Alkoxy substituiertes $C_1$-$C_6$-Alkyl, jeweils gegebenenfalls durch Cyano oder Halogen substituiertes $C_3$-$C_6$-Alkenyl oder $C_3$-$C_6$-Alkinyl, oder gegebenenfalls durch Cyano, Halogen oder $C_1$-$C_4$-Alkyl substituiertes $C_3$-$C_6$-Cycloalkyl steht,

$R^{26}$ für Wasserstoff, gegebenenfalls durch Cyano, Hydroxy, Halogen oder $C_1$-$C_4$-Alkoxy substituiertes $C_1$-$C_6$-Alkyl, jeweils gegebenenfalls durch Cyano oder Halogen substituiertes $C_3$-$C_6$-Alkenyl oder $C_3$-$C_6$-Alkinyl, gegebenenfalls durch Cyano, Halogen oder $C_1$-$C_4$-Alkyl substituiertes $C_3$-$C_6$-Cycloalkyl, oder gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Phenyl steht, oder zusammen mit $R^{25}$ für jeweils gegebenenfalls durch $C_1$-$C_4$-Alkyl substituiertes $C_2$-$C_6$-Alkandiyl oder $C_2$-$C_5$-Oxaalkandiyl steht,

$X^4$ für Nitro, Cyano, Carboxy, Carbamoyl, Formyl, Sulfamoyl, Hydroxy, Amino, Halogen, $C_1$-$C_4$-Alkyl, $C_1$-$C_4$-Halogenalkyl, $C_1$-$C_4$-Alkoxy oder $C_1$-$C_4$-Halogenalkoxy steht, und

$X^5$ für Nitro, Cyano, Carboxy, Carbamoyl, Formyl, Sulfamoyl, Hydroxy, Amino, Halogen, $C_1$-$C_4$-Alkyl, $C_1$-$C_4$-Halogenalkyl, $C_1$-$C_4$-Alkoxy oder $C_1$-$C_4$-Halogenalkoxy steht.

Es wurde weiterhin gefunden, dass Zusammensetzungen enthaltend eine Phase enthaltend mindestens einen gelösten Wirkstoff der Formeln (I) oder (I')

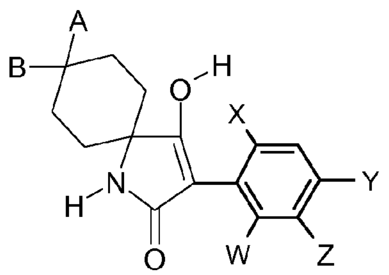

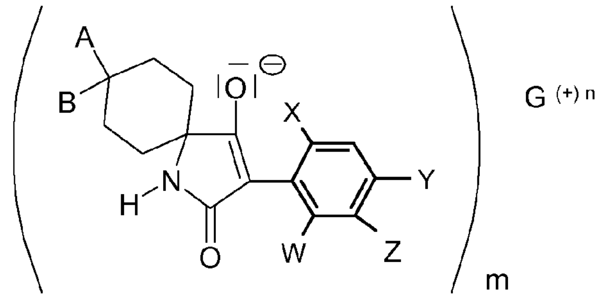

(I')　　　　(I)

in welchen

A, B, G, m, n, W, X, Y und Z die oben angegebene Bedeutung haben

einen schnelleren Wirkeintritt und/oder eine besserere Kulturpflanzenverträglichkeit und/oder eine höhere Wirksamkeit aufweisen als beispielsweise entsprechende SC-Formulierungen, in denen insbesondere Wirkstoffe der Formel (I') partikulär vorliegen, solange die Formulierung nicht in Wasser verdünnt wird. In den erfindungsgemäßen Formulierungen liegen die Wirkstoffe der Formel (I) bereits in den konzentrierten Zusammensetzungen in gelöster Form vor.

Dies ist besonders überraschend, weil aufgrund der relativ hohen Wasserlöslichkeit der Wirkstoffe Formel (I') ausgehend von jedem Formuliertyp nach Verdünnen in Wasser eine sofortige Verfügbarkeit - und damit eine Unabhängigkeit der Wirkung vom ursprünglichen Formulierungstyp - zu erwarten gewesen wäre.

Gegenstand der vorliegenden Erfindung sind demnach Zusammensetzungen enthaltend mindestens ein Lösemittel und mindestens eine Verbindung der Formel (I) oder (I') in gelöster Form.

Gegenstand der vorliegenden Erfindung sind ebenfalls Verfahren zur Herstellung von wasserlöslichen Konzentraten enthaltend mindestens eine Verbindung der Formel (I) oder (I').

Gegenstand der vorliegenden Erfindung ist außerdem die Verwendung von Zusammensetzungen enthaltend eine Phase enthaltend mindestens eine Verbindung der Formel (I) oder (I') in gelöster Form zur Bekämpfung von unerwünschtem Pflanzenwuchs und/oder tierischen Schädlingen.

Im Allgemeinen sind die oben aufgeführten Verbindungen der Formeln (I) oder (I') erfindungsgemäß einsetzbar.

Erfindungsgemäß bevorzugte Verbindungen der Formel (I) sind die bei den Herstellungsbeispielen aufgeführten Verbindungen, wobei jede dort enthaltene Verbindung für sich bevorzugt ist.

Erfindungsgemäß bevorzugte Verbindungen der Formel (I') sind in Tabelle A aufgezeigt, wobei jede dort enthaltene Verbindung für sich bevorzugt ist.

**Tabelle A**

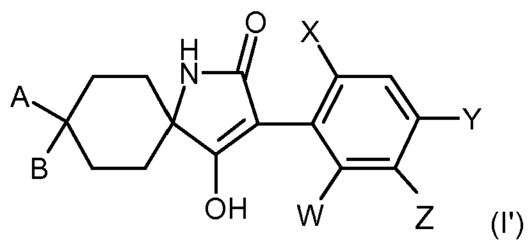

(I') mit Z=H

| Bsp.-Nr. | W | X | Y | A | B | Bekannt aus WO 06/089633; Bsp.-Nr. |
|---|---|---|---|---|---|---|
| I'-1 | $CH_3$ | $CH_3$ | $CH_3$ | -O-$(CH_2)_2$-O- | | I-1-a-2 |
| I'-2 | $CH_3$ | $CH_3$ | Cl | -O-$(CH_2)_2$-O- | | I-1-a-4 |
| I'-3 | $CH_3$ | $CH_3$ | Br | -O-$(CH_2)_2$-O- | | I-1-a-26 |

(fortgesetzt)

| Bsp.-Nr. | W | X | Y | A | B | Bekannt aus WO 06/089633; Bsp.-Nr. |
|---|---|---|---|---|---|---|
| I'-4 | $CH_3$ | $CH_3$ | $CH_3$ | $-O-(CH_2)_3-O-$ | | I-1-a-18 |
| I'-5 | $CH_3$ | $CH_3$ | Cl | $-O-(CH_2)_3-O-$ | | I-1-a-14 |
| I'-6 | $CH_3$ | $CH_3$ | Br | $-O-(CH_2)_3-O-$ | | I-1-a-19 |

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:

W steht bevorzugt für Wasserstoff, Chlor, Brom, $C_1$-$C_4$-Alkyl, $C_1$-$C_4$-Alkoxy, $C_1$-$C_2$-Halogenalkyl oder $C_1$-$C_2$-Halogenalkoxy,

X steht bevorzugt für Chlor, Brom, $C_1$-$C_4$-Alkyl, $C_1$-$C_4$-Alkoxy, $C_1$-$C_4$-Halogenalkyl, $C_1$-$C_4$-Halogenalkoxy oder Cyano,

Y und Z stehen bevorzugt unabhängig voneinander für Wasserstoff, Fluor, Chlor, Brom, $C_1$-$C_4$-Alkyl, $C_1$-$C_6$-Alkoxy, $C_1$-$C_4$-Halogenalkyl, $C_1$-$C_4$-Halogenalkoxy oder Cyano,

A und B und das Kohlenstoffatom, an dass sie gebunden sind, stehen besonders bevorzugt für ein gegebenenfalls einfach bis dreifach durch $C_1$-$C_4$-Alkyl, $C_1$-$C_3$-Halogenalkyl, $C_1$-$C_4$-Alkoxy oder $C_1$-$C_4$-Alkoxy-$C_1$-$C_2$-alkyl substituiertes fünf- oder sechsgliedriges Ketal,

G steht bevorzugt für Lithium, Natrium, Kalium, Cäsium, Magnesium-Halogen-Kationen, Magnesium, Calcium oder einem Ammoniumion

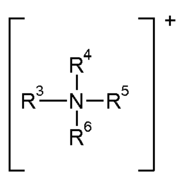
,

in welchem $R^3$, $R^4$, $R^5$, $R^6$ die oben genannte Bedeutung haben.

In den als besonders bevorzugt genannten Restedefinitionen steht Halogen für Fluor, Chlor und Brom, insbesondere für Fluor und Chlor.

W steht besonders bevorzugt für Wasserstoff, Chlor, Brom, Methyl, Ethyl, Methoxy, Ethoxy oder Trifluormethyl,

X steht besonders bevorzugt für Chlor, Brom, Methyl, Ethyl, Methoxy, Ethoxy, Trifluormethyl, Difluormethoxy, Trifluormethoxy oder Cyano,

Y und Z stehen besonders bevorzugt unabhängig voneinander für Wasserstoff, Fluor, Chlor, Brom, Methyl, Ethyl, Methoxy, Trifluormethyl, Trifluormethoxy oder Cyano,

A und B und das Kohlenstoffatom, an dass sie gebunden sind, stehen besonders bevorzugt für ein gegebenenfalls einfach bis zweifach durch Methyl, Ethyl, Propyl, Trifluormethyl, Monochlormethyl, Methoxy, Ethoxy, Methoxymethyl oder Ethoxymethyl substituiertes fünfoder sechsgliedriges Ketal,

G steht besonders bevorzugt für Lithium, Natrium, Kalium, Cäsium, Magnesium-chlorid-kation, Magnesium-bromid-kation, Magnesium-jodid-kation, Magnesium, Calcium oder

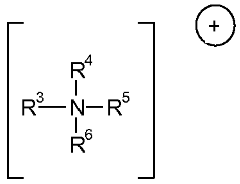

,

einem Ammoniumion in welchem $R^3$, $R^4$, $R^5$, $R^6$ unabhängig voneinander besonders bevorzugt für Wasserstoff $C_1$-$C_6$-Alkyl oder Benzyl steht.

m steht besonders bevorzugt für die Zahlen 1 oder 2,

n steht besonders bevorzugt für die Zahlen 1 oder 2,

W steht ganz besonders bevorzugt für Wasserstoff, Chlor, Brom, Methyl, Ethyl oder Methoxy,

X steht ganz besonders bevorzugt für Chlor, Brom, Methyl, Ethyl, Methoxy oder Ethoxy,

Y und Z stehen ganz besonders bevorzugt unabhängig voneinander für Wasserstoff, Chlor, Brom oder Methyl,

A und B und das Kohlenstoffatom, an dass sie gebunden sind, stehen ganz besonders bevorzugt für ein gegebenenfalls einfach bis zweifach durch Methyl, Ethyl, Propyl, Monochlormethyl oder Methoxymethyl substituiertes fünf- oder sechsgliedriges Ketal,

G steht ganz besonders bevorzugt für Lithium, Natrium, Kalium, Cäsium, Magnesium-bromidkation, Magnesium, Calcium oder einem Ammoniumion

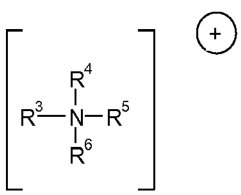

,

in welchem $R^3$, $R^4$, $R^5$, $R^6$ unabhängig voneinander ganz besonders bevorzugt für $C_1$-$C_4$-Alkyl oder Benzyl stehen,

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Hervorgehoben sind Verbindungen der Formel (I) in welchen G für Wasserstoff steht.

Im Fall der Verbindungen der Formel (I) ist der Phenylring hervorgehoben 3-fach substituiert, wobei sich folgende Substitutionsmuster ergeben: 2,4,6-; 2,4,5- oder 2,5,6-Substitution. Außerdem ist im der Fall der Verbindungen der Formel (I) der Phenylring hervorgehoben 4-fach substituiert, wobei sich folgendes Substitutionsmuster ergibt: 2,4,5,6-Substitution. Im Falle der Verbindungen der Formel (I) ist der Phenylring hervorgehoben auch 2-fach (2,4-; 2,5-Postition) substituiert. Im Falle der Verbindungen der Formel (I) ist der Phenylring hervorgehoben auch 1-fach (ortho Postition)

substituiert. Die übrigen Substituenten W, X, Y, Z, G, A und B haben die im Text genannten Definitionen.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im Einzelnen seien außer den bei den Herstellungsbeispielen genannten Verbindungen die folgenden Verbindungen der Formel (I) genannt:

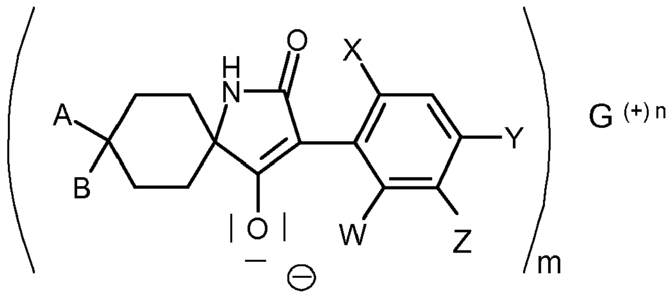

(I)

Tabelle 1: $G^{(+)n} = Na^+$; m = 1; Z=H

| W | X | Y | A | B |
|---|---|---|---|---|
| $CH_3$ | $CH_3$ | $CH_3$ | $-O-(CH_2)_2-O-$ | |
| $CH_3$ | $CH_3$ | Cl | $-O-(CH_2)_2-O-$ | |
| $CH_3$ | $CH_3$ | Br | $-O-(CH_2)_2-O-$ | |
| $CH_3$ | $CH_3$ | $CH_3$ | $-O-(CH_2)_3-O-$ | |
| $CH_3$ | $CH_3$ | Cl | $-O-(CH_2)_3-O-$ | |
| $CH_3$ | $CH_3$ | Br | $-O-(CH_2)_3-O-$ | |

**Tabelle 2:**

A, B, W, X und Y wie in Tabelle 1 angegeben

$$G^{(+)}{}_n = K^+;\ m = 1$$

**Tabelle 3:**

A, B, W, X und Y wie in Tabelle 1 angegeben

$$G^{(+)}{}_n = Li^+;\ m = 1$$

**Tabelle 4:**

A, B, W, X und Y wie in Tabelle 1 angegeben

(fortgesetzt)

$$G^{(+)}{}_{n} = Mg^{2+};\ m = 2$$

**Tabelle 5:**

A, B, W, X und Y wie in Tabelle 1 angegeben

$$G^{(+)}{}_{n} = Ca^{2+};\ m = 2$$

**Tabelle 6:**

A, B, W, X und Y wie in Tabelle 1 angegeben

$$G^{(+)}{}_{n} = [H_2N(CH_3)_2]^{+};\ m = 1$$

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.

n steht bevorzugt für die Zahlen 0, 1, 2, 3 oder 4.

$A^1$ steht bevorzugt für eine der nachstend skizzierten divalenten heterocyclischen Gruppierungen

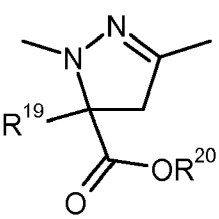 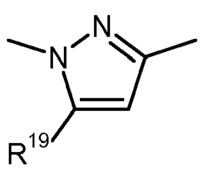 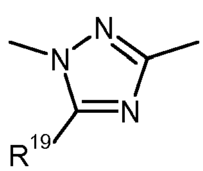 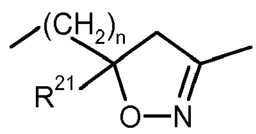 .

$A^2$ steht bevorzugt für jeweils gegebenenfalls durch Methyl, Ethyl, Methoxycarbonyl oder Ethoxycarbonyl substituiertes Methylen oder Ethylen.

$R^{14}$ steht bevorzugt für Hydroxy, Mercapto, Amino, Methoxy, Ethoxy, n- oder i-Propoxy, n-, i-, s-oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.

$R^{15}$ steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.

$R^{16}$ steht bevorzugt für jeweils gegebenenfalls durch Fluor, Chlor und/oder Brom substituiertes Methyl, Ethyl, n- oder i-Propyl.

$R^{17}$ steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl,

Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.

$R^{18}$ steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furyl-methyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit $R^{17}$ für einen der Reste $-CH_2-O-CH_2-CH_2-$ und $-CH_2-CH_2-O-CH_2-CH_2-$, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.

$R^{19}$ steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.

$R^{20}$ steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.

$R^{21}$ steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t-Butyl, Cyclopropyl, Cyclobutyl, Cyclopentyl, Cyclohexyl oder Phenyl.

$X^1$ steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.

$X^2$ steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.

$X^3$ steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.

$R^{22}$ steht bevorzugt für Wasserstoff, Methyl, Ethyl, n- oder i-Propyl.

$R^{23}$ steht bevorzugt für Wasserstoff, Methyl, Ethyl, n- oder i-Propyl.

$R^{24}$ steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclo-butylamino, Cyclopentylamino oder Cyclohexylamino.

$R^{25}$ steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.

$R^{26}$ steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, oder zusammen mit $R^{25}$ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.

$X^4$ steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

$X^5$ steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

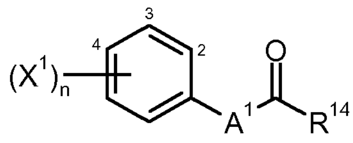
(IIa)

Tabelle Beispiele für die Verbindungen der Formel (IIa)

| Beispiel- Nr. | (Positionen) $(X^1)_n$ | $A^1$ | $R^{14}$ |
|---|---|---|---|
| IIa-1 | (2) Cl, (4) Cl | 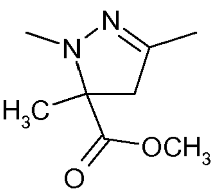 | $OCH_3$ |
| IIa-2 | (2) C1, (4) Cl | 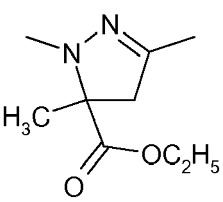 | $OCH_3$ |
| IIa-3 | (2) Cl, (4) Cl | 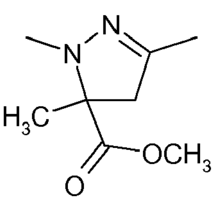 | $OC_2H_5$ |
| IIa-4 | (2) Cl, (4) Cl | 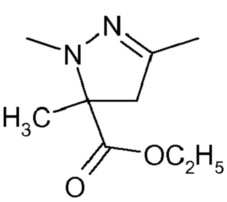 | $OC_2H_5$ |

(fortgesetzt)

| **Beispiel- Nr.** | **(Positionen) $(X^1)_n$** | **$A^1$** | **$R^{14}$** |
|---|---|---|---|
| IIa-5 | (2) C1 | 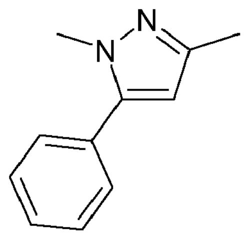 | $OCH_3$ |
| IIa-6 | (2) Cl, (4) Cl | 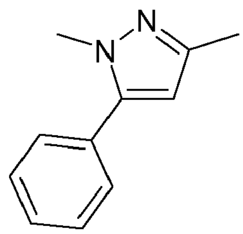 | $OCH_3$ |
| IIa-7 | (2) F | 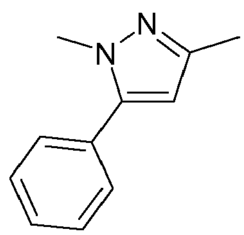 | $OCH_3$ |
| IIa-8 | (2) F | 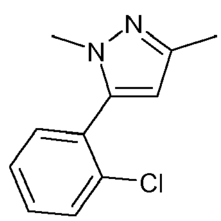 | $OCH_3$ |
| IIa-9 | (2) Cl, (4) Cl | 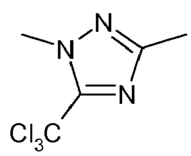 | $OC_2H_5$ |
| IIa-10 | (2) C1, (4) $CF_3$ | 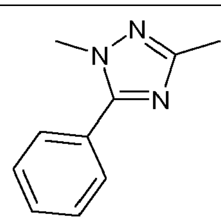 | $OCH_3$ |
| IIa-11 | (2) C1 | 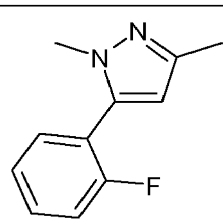 | $OCH_3$ |

(fortgesetzt)

| Beispiel- Nr. | (Positionen) $(X^1)_n$ | $A^1$ | $R^{14}$ |
|---|---|---|---|
| IIa-12 | - | 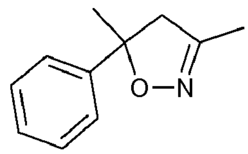 | $OC_2H_5$ |
| IIa-13 | (2) C1, (4) C1 | 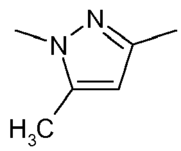 | $OC_2H_5$ |
| IIa-14 | (2) C1, (4) C1 | 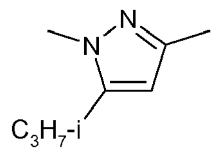 | $OC_2H_5$ |
| IIa-15 | (2) C1, (4) C1 | 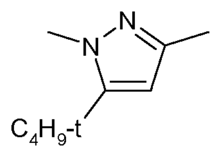 | $OC_2H_5$ |
| IIa-16 | (2) C1, (4) C1 | 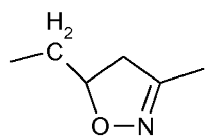 | $OC_2H_5$ |
| IIa-17 | (2) C1, (4) C1 | 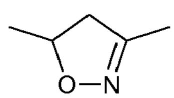 | $OC_2H_5$ |
| IIa-18 | - | 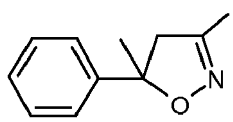 | OH |

Beispiele für die als erfindungsgemäße Herbizid-Safener ganz besonders bevorzugten Verbindungen der Formel (IIb) sind in der nachstehenden Tabelle aufgeführt.

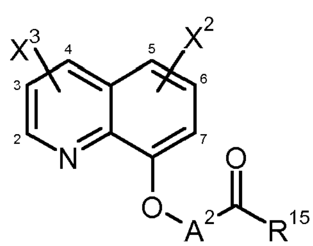

(IIb)

**Tabelle** Beispiele für die Verbindungen der Formel (IIb)

| Beispiel-Nr. | (Position) $X^2$ | (Position) $X^3$ | $A^2$ | $R^{15}$ |
|---|---|---|---|---|
| IIb-1 | (5) C1 | - | $CH_2$ | OH |
| IIb-2 | (5) C1 | - | $CH_2$ | $OCH_3$ |
| IIb-3 | (5) C1 | - | $CH_2$ | $OC_2H_5$ |
| IIb-4 | (5) C1 | - | $CH_2$ | $OC_3H_7$-n |
| IIb-5 | (5) C1 | - | $CH_2$ | $OC_3H_7$-i |
| IIb-6 | (5) C1 | - | $CH_2$ | $OC_4H_9$-n |
| IIb-7 | (5) C1 | - | $CH_2$ | $OCH(CH_3)C_5H_{11}$-n |
| IIb-8 | (5) C1 | (2) F | $CH_2$ | OH |
| IIb-9 | (5) C1 | (2) C1 | $CH_2$ | OH |
| IIb-10 | (5) C1 | - | $CH_2$ | $OCH_2CH=CH_2$ |
| IIb-11 | (5) C1 | - | $CH_2$ | $OC_4H_9$-i |
| IIb-12 | (5) C1 | - | $CH_2$ | 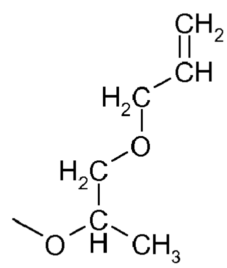 |
| IIb-13 | (5) C1 | - | 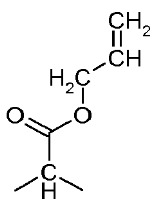 | $OCH_2CH=CH_2$ |
| IIb-14 | (5) C1 | - | 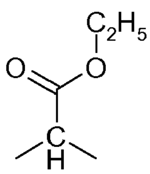 | $OC_2H_5$ |
| IIb-15 | (5) C1 | - | 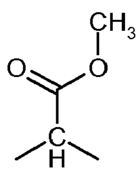 | $OCH_3$ |

Beispiele für die als erfindungsgemäße Herbizid-Safener ganz besonders bevorzugten Verbindungen der Formel (IIc) sind in der nachstehenden Tabelle aufgeführt.

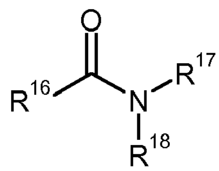

(IIc)

**Tabelle** Beispiele für die Verbindungen der Formel (IIc)

| **Beispiel-Nr.** | **$R^{16}$** | **$N(R^{17},R^{18})$** |
|---|---|---|
| IIc-1 | $CHCl_2$ | $N(CH_2CH=CH_2)_2$ |
| IIc-2 | $CHCl_2$ | 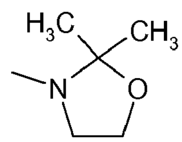 |
| IIc-3 | $CHCl_2$ | 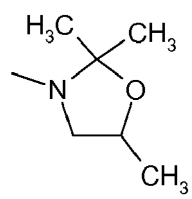 |
| IIc-4 | $CHCl_2$ | 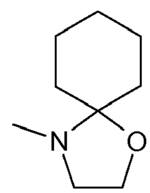 |
| IIc-5 | $CHCl_2$ | 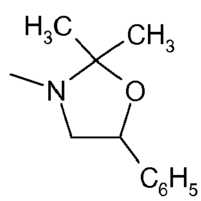 |
| IIc-6 | $CHCl_2$ | 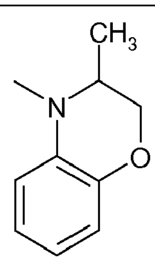 |
| IIc-7 | $CHCl_2$ | 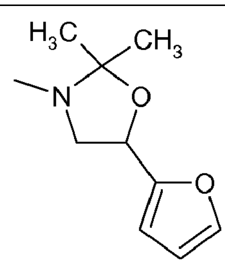 |

Beispiele für die als erfindungsgemäße Herbizid-Safener ganz besonders bevorzugten Verbindungen der Formel (IId) sind in der nachstehenden Tabelle aufgeführt.

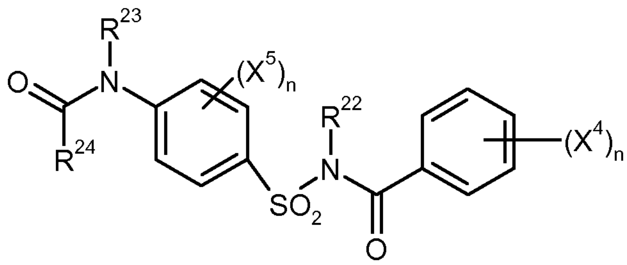

(IId)

**Tabelle** Beispiele für die Verbindungen der Formel (IId)

| Beispiel-Nr. | $R^{22}$ | $R^{23}$ | $R^{24}$ | (Positionen) $(X^4)_n$ | (Positionen) $(X^5)_n$ |
|---|---|---|---|---|---|
| IId-1 | H | H | $CH_3$ | (2) $OCH_3$ | - |
| IId-2 | H | H | $C_2H_5$ | (2) $OCH_3$ | - |
| IId-3 | H | H | $C_3H_7$-n | (2) $OCH_3$ | - |
| IId-4 | H | H | $C_3H_7$-i | (2) $OCH_3$ | - |
| IId-5 | H | H | 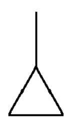 | (2) $OCH_3$ | - |
| IId-6 | H | H | $CH_3$ | (2) $OCH_3$ (5) $CH_3$ | - |
| IId-7 | H | H | $C_2H_5$ | (2) $OCH_3$ (5) $CH_3$ | - |
| IId-8 | H | H | $C_3H_7$-n | (2) $OCH_3$ (5) $CH_3$ | - |
| IId-9 | H | H | $C_3H_7$-i | (2) $OCH_3$ (5) $CH_3$ | - |
| IId-10 | H | H | 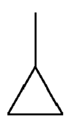 | (2) $OCH_3$ (5) $CH_3$ | - |
| IId-11 | H | H | $OCH_3$ | (2) $OCH_3$ (5) $CH_3$ | - |
| IId-12 | H | H | $OC_2H_5$ | (2) $OCH_3$ (5) $CH_3$ | - |
| IId-13 | H | H | $OC_3H_7$-i | (2) $OCH_3$ (5) $CH_3$ | - |
| IId-14 | H | H | $SCH_3$ | (2) $OCH_3$ (5) $CH_3$ | - |
| IId-15 | H | H | $SC_2H_5$ | (2) $OCH_3$ (5) $CH_3$ | - |
| IId-16 | H | H | $SC_3H_7$-i | (2) $OCH_3$ (5) $CH_3$ | - |
| IId-17 | H | H | $NHCH_3$ | (2) $OCH_3$ (5) $CH_3$ | - |
| IId-18 | H | H | $NHC_2H_5$ | (2) $OCH_3$ (5) $CH_3$ | - |
| IId-19 | H | H | $NHC_3H_7$-i | (2) $OCH_3$ (5) $CH_3$ | - |
| IId-20 | H | H | 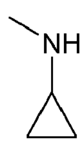 | (2) $OCH_3$ (5) $CH_3$ | - |

(fortgesetzt)

| Beispiel-Nr. | $R^{22}$ | $R^{23}$ | $R^{24}$ | (Positionen) $(X^4)_n$ | (Positionen) $(X^5)_n$ |
|---|---|---|---|---|---|
| IId-21 | H | H | $NHCH_3$ | (2) $OCH_3$ | - |
| IId-22 | H | H | $NHC_3H_7$-i | (2) $OCH_3$ | - |
| IId-23 | H | H | $N(CH_3)_2$ | (2) $OCH_3$ | - |
| IId-24 | H | H | $N(CH_3)_2$ | (3) $CH_3$ (4) $CH_3$ | - |
| IId-25 | H | H | $CH_2$-O-$CH_3$ | (2) $OCH_3$ | - |

Beispiele für die als erfindungsgemäße Herbizid-Safener ganz besonders bevorzugten Verbindungen der Formel (IIe) sind in der nachstehenden Tabelle aufgeführt.

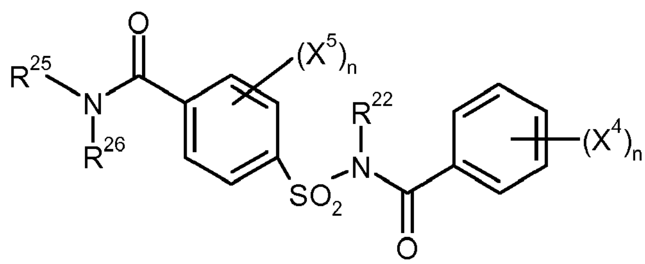

(IIe)

**Tabelle** Beispiele für die Verbindungen der Formel (IIe)

| Beispiel-Nr. | $R^{22}$ | $R^{25}$ | $R^{26}$ | (Positionen) $(X^4)_n$ | (Positionen) $(X^5)_n$ |
|---|---|---|---|---|---|
| IIe-1 | H | H | $CH_3$ | (2) $OCH_3$ | - |
| IIe-2 | H | H | $C_2H_5$ | (2) $OCH_3$ | - |
| IIe-3 | H | H | $C_3H_7$-n | (2) $OCH_3$ | - |
| IIe-4 | H | H | $C_3H_7$-i | (2) $OCH_3$ | - |
| IIe-5 | H | H | 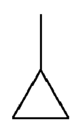 | (2) $OCH_3$ | - |
| IIe-6 | H | $CH_3$ | $CH_3$ | (2) $OCH_3$ | - |
| IIe-7 | H | H | $CH_3$ | (2) $OCH_3$ (5) $CH_3$ | - |
| IIe-8 | H | H | $C_2H_5$ | (2) $OCH_3$ (5) $CH_3$ | - |
| IIe-9 | H | H | $C_3H_7$-n | (2) $OCH_3$ (5) $CH_3$ | - |
| IIe-10 | H | H | $C_3H_7$-i | (2) $OCH_3$ (5) $CH_3$ | - |
| IIe-11 | H | H | 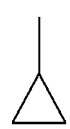 | (2) $OCH_3$ (5) $CH_3$ | - |
| IIe-12 | H | $CH_3$ | $CH_3$ | (2) $OCH_3$ (5) $CH_3$ | - |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und

die Verbindungen IIe-5 und IIe-11 am meisten bevorzugt, wobei Cloquintocet-mexyl und Mefenpyr-diethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

Tabelle Beispiele für die erfindungsgemäßen Kombinationen

| Wirkstoffe der Formel (I) | Safener |
|---|---|
| A.1 | Cloquintocet-mexyl |
| A.1 | Fenchlorazole-ethyl |
| A.1 | Isoxadifen-ethyl |
| A.1 | Mefenpyr-diethyl |
| A.1 | Furilazole |
| A.1 | Fenclorim |
| A.1 | Cumyluron |
| A.1 | Daimuron /Dymron |
| A.1 | Dimepiperate |
| A.1 | IIe-11 |
| A.1 | IIe-5 |
| A.2 | Cloquintocet-mexyl |
| A.2 | Fenchlorazole-ethyl |
| A.2 | Isoxadifen-ethyl |
| A.2 | Mefenpyr-diethyl |
| A.2 | Furilazole |
| A.2 | Fenclorim |
| A.2 | Cumyluron |
| A.2 | Daimuron /Dymron |
| A.2 | Dimepiperate |
| A.2 | IIe-11 |
| A.2 | IIe-5 |
| A.3 | Cloquintocet-mexyl |
| A.3 | Fenchlorazole-ethyl |
| A.3 | Isoxadifen-ethyl |
| A.3 | Mefenpyr-diethyl |
| A.3 | Furilazole |
| A.3 | Fenclorim |

(fortgesetzt)

| Wirkstoffe der Formel (I) | Safener |
|---|---|
| A.3 | Cumyluron |
| A.3 | Daimuron /Dymron |
| A.3 | Dimepiperate |
| A.3 | IIe-5 |
| A.3 | IIe-11 |
| A.4 | Cloquintocet-mexyl |
| A.4 | Fenchlorazole-ethyl |
| A.4 | Isoxadifen-ethyl |
| A.4 | Mefenpyr-diethyl |
| A.4 | Furilazole |
| A.4 | Fenclorim |
| A.4 | Cumyluron |
| A.4 | Daimuron /Dymron |
| A.4 | Dimepiperate |
| A.4 | IIe-11 |
| A.4 | IIe-5 |
| A.5 | Cloquintocet-mexyl |
| A.5 | Fenchlorazole-ethyl |
| A.5 | Isoxadifen-ethyl |
| A.5 | Mefenpyr-diethyl |
| A.5 | Furilazole |
| A.5 | Fenclorim |
| A.5 | Cumyluron |
| A.5 | Daimuron /Dymron |
| A.5 | Dimepiperate |
| A.5 | IIe-5 |
| A.5 | IIe-11 |
| A.6 | Cloquintocet-mexyl |
| A.6 | Fenchlorazole-ethyl |
| A.6 | Isoxadifen-ethyl |
| A.6 | Mefenpyr-diethyl |
| A.6 | Furilazole |
| A.6 | Fenclorim |
| A.6 | Cumyluron |
| A.6 | Daimuron /Dymron |
| A.6 | Dimepiperate |
| A.6 | IIe-5 |
| A.6 | IIe-11 |

(fortgesetzt)

| Wirkstoffe der Formel (I) | Safener |
|---|---|
| A.7 | Cloquintocet-mexyl |
| A.7 | Fenchlorazole-ethyl |
| A.7 | Isoxadifen-ethyl |
| A.7 | Mefenpyr-diethyl |
| A.7 | Furilazole |
| A.7 | Fenclorim |
| A.7 | Cumyluron |
| A.7 | Daimuron /Dymron |
| A.7 | Dimepiperate |
| A.7 | IIe-5 |
| A.7 | IIe-11 |
| A.8 | Cloquintocet-mexyl |
| A.8 | Fenchlorazole-ethyl |
| A.8 | Isoxadifen-ethyl |
| A.8 | Mefenpyr-diethyl |
| A.8 | Furilazole |
| A.8 | Fenclorim |
| A.8 | Cumyluron |
| A.8 | Daimuron /Dymron |
| A.8 | Dimepiperate |
| A.8 | IIe-5 |
| A.8 | IIe-11 |

Es wurde nun überraschend gefunden, dass die oben definierten Wirkstoffkombinationen aus 4'4'-Dioxaspiro-spirocyclisch substituierten Tetramate der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von 4'4'-Dioxaspiro-spirocyclisch substituierten Tetramate auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Alle in den erfindungsgemäßen Mitteln enthaltenen Wirkstoffe der Formel (I) können nach im Stand der Technik beschriebenen Verfahren (siehe oben genannte Referenzen) hergestellt werden. Ihre Wirkung ist gut, jedoch insbesondere bei niedrigen Aufwandmengen und Konzentrationen nicht immer voll zufriedenstellend. Weiterhin ist die Pflanzenverträglichkeit dieser Verbindungen nicht immer ausreichend. Es besteht deshalb ein Bedarf für eine Wirkungssteigerung und/oder Verbesserung der Pflanzenverträglichkeit gegenüber Kulturpflanzen der die Verbindungen enthaltenden Pflanzenschutzmittel.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des

Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. Eine Wirkungssteigerung z.B. durch Ammoniumsulfat ist beispielsweise für die Herbizide Glyphosat und Phosphinothricin beschrieben (US 6 645 914, EP-A2 0 036 106).

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Herbizide bzw. insektizide Zusammensetzungen von cyclischen Ketoenolen mit Ammonium- oder Phosphoniumsalzen zur Wirkstoffsteigerung sind beschrieben in WO 07/068427 und WO 07/068428.

Es wurde nun überraschend gefunden, dass sich die Wirkung von Herbiziden und/oder Akariziden und/oder Insektiziden aus der Klasse der 4'4'-Dioxaspiro-spirocyclisch substituierten Tetramate durch den Zusatz von Ammonium- oder Phosphoniumsalzen zur Anwendungslösung oder durch den Einbau dieser Salze in eine Formulierung enthaltend 4'4'-Dioxaspiro-spirocyclisch substituierten Tetramate, deutlich steigern lässt. Gegenstand der vorliegenden Erfindung ist also die Verwendung von Ammonium- oder Phosphoniumsalzen zur Wirkungssteigerung von Pflanzenschutzmitteln, die insektizid und/oder akarizid und/oder herbizid wirksame 4'4'-Dioxaspiro-spirocyclisch substituierte Tetramate als Wirkstoff enthalten. Gegenstand der Erfindung sind ebenfalls Mittel, die insektizid und/oder akarizid und/oder herbizid wirksame 4'4'-Dioxaspiro-spirocyclisch substituierte Tetramate und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Insekten und/oder Spinnmilben und/oder unerwünschtem Pflanzenwuchs.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend 4'4'-Dioxaspiro-spirocyclisch substituierte Tetramate steigern, werden durch Formel (II') definiert

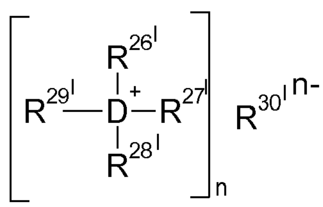

(II')

in welcher

| | |
|---|---|
| D | für Stickstoff oder Phosphor steht, |
| D | bevorzugt für Stickstoff steht, |
| $R^{26'}$, $R^{27'}$, $R^{28'}$ und $R^{29'}$ | unabhängig voneinander für Wasserstoff oder jeweils gegebenenfalls substituiertes $C_1$-$C_8$-Alkyl oder einfach oder mehrfach ungesättigtes, gegebenenfalls substituiertes $C_1$-$C_8$-Alkylen stehen, wobei die Substituenten aus Halogen, Nitro und Cyano ausgewählt sein können, |
| $R^{26'}$, $R^{27'}$, $R^{28'}$ und $R^{29'}$ | bevorzugt unabhängig voneinander für Wasserstoff oder jeweils gegebenenfalls substituiertes $C_1$-$C_4$-Alkyl stehen, wobei die Substituenten aus Halogen, Nitro und Cyano ausgewählt sein können, |
| $R^{26'}$, $R^{27'}$, $R^{28'}$ und $R^{29'}$ | besonders bevorzugt unabhängig voneinander für Wasserstoff, Methyl, Ethyl, n-Propyl, i-Propyl, n-Butyl, i-Butyl, s-Butyl oder t-Butyl stehen, |
| $R^{26'}$ $R^{27'}$, $R^{28'}$ und $R^{29'}$ | ganz besonders bevorzugt für Wasserstoff stehen, |
| $R^{26'}$, $R^{27'}$, $R^{28'}$ und $R^{29'}$ | weiterhin ganz besonders bevorzugt gleichzeitig für Methyl oder gleichzeitig für Ethyl stehen, |
| n | für 1, 2, 3 oder 4 steht, |

| | |
|---|---|
| n | bevorzugt für 1 oder 2 steht, |
| $R^{30'}$ | für ein anorganisches oder organisches Anion steht, |
| $R^{30'}$ | bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat, Citrat oder Oxalat steht, |
| $R^{30'}$ | weiterhin bevorzugt für Carbonat, Pentaborat, Sulfit, Benzoat, Hydrogenoxalat, Hydrogencitrat, Methylsulfat oder Tetrafluoroborat steht, |
| $R^{30'}$ | besonders bevorzugt für Laktat, Sulfat, Nitrat, Thiosulfat, Thiocyanat, Citrat, Oxalat oder Formiat steht, |
| $R^{30'}$ | außerdem besonders bevorzugt für Acetat, Monohydrogenphosphat oder Dihydrogenphosphat steht und |
| $R^{30'}$ | ganz besonders bevorzugt für Sulfat, Thiocyanat, Dihydrogenphosphat oder Monohydrogenphosphat steht. |

Die Ammonium- und Phosphoniumsalze der Formel (II') können in einem breiten Konzentrationsbereich zur Steigerung der Wirkung von Pflanzenschutzmitteln enthaltend Verbindungen der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die herbizid wirksame 4'4'-Dioxaspiro-spirocyclisch substituierte Tetramate als Wirkstoff enthalten. Gegenstand der Erfindung sind ebenfalls Mittel, die insektizid und/oder akarizid und/oder herbizid wirksame 4'4'-Dioxaspiro-spirocyclisch substituierte Tetramate, Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Insekten und / oder Spinnmilben und / oder unerwünschtem Pflanzenwuchs.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel

$$R\text{-}O\text{-}(\text{-}AO)_v\text{-}R' \qquad (III')$$

in welcher

| | |
|---|---|
| R | für geradkettiges oder verzweigtes Alkyl mit 4 bis 20 Kohlenstoffatomen steht, |
| R' | für Wasserstoff, Methyl, Ethyl, n-Propyl, i-Propyl, n-Butyl, i-Butyl, t-Butyl, n-Pentyl oder n-Hexyl steht, |
| AO | für einen Ethylenoxid-Rest, einen Propylenoxid-Rest, einen Butylenoxid-Rest oder für Gemische aus Ethylenoxid- und Propylenoxid-Resten oder Butylenoxid-Resten steht und |

v für Zahlen von 2 bis 30 steht.

Dabei werden solche Alkanol-Alkoxylate, bei denen R' Wasserstoff ist, als "offene" Alkanol-Alkoxylate bezeichnet. Eine bevorzugte Gruppe von Penetrationsförderern sind Alkanolalkoxylate der Formel

$$R\text{-}O\text{-}(\text{-}EO\text{-})_n\text{-}R' \qquad \text{(III'-a)}$$

in welcher

R die oben angegebene Bedeutung hat,
R' die oben angegebene Bedeutung hat,
EO für $-CH_2-CH_2-O-$ steht und
n für Zahlen von 2 bis 20 steht.

Eine weitere bevorzugte Gruppe von Penetrationsförderern sind Alkanol-alkoxylate der Formel

$$R\text{-}O\text{-}(\text{-}EO\text{-})_p\text{-}(\text{-}PO\text{-})_q\text{-}R' \qquad \text{(III'-b)}$$

in welcher

R die oben angegebene Bedeutung hat,
R' die oben angegebene Bedeutung hat,
EO für $-CH_2-CH_2-O-$ steht,
PO für

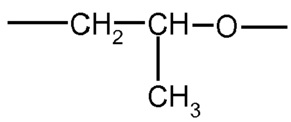

steht,
p für Zahlen von 1 bis 10 steht und
q für Zahlen von 1 bis 10 steht.

Eine weitere bevorzugte Gruppe von Penetrationsförderern sind Alkanol-Alkoxylate der Formel

$$R\text{-}O\text{-}(\text{-}PO\text{-})_r\text{-}(EO\text{-})_S\text{-}R' \qquad \text{(III'-c)}$$

in welcher

R die oben angegebene Bedeutung hat,
R' die oben angegebene Bedeutung hat,
EO für $-CH_2-CH_2-O-$ steht,
PO für

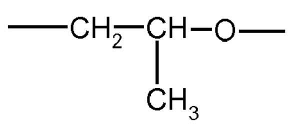

steht,

Eine weitere bevorzugte Gruppe von Penetrationsförderern sind Alkanol-alkoxylate der Formel

$$R\text{-}O\text{-}(\text{-}EO\text{-})_p\text{-}(\text{-}BO\text{-})_q\text{-}R' \qquad (III'\text{-}d)$$

in welcher

R und R' die oben angegebenen Bedeutungen haben,
EO für $CH_2$-$CH_2$-O- steht,
BO für

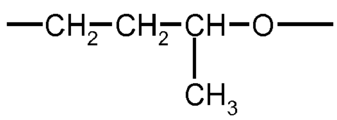

steht,
p für Zahlen von 1 bis 10 steht und
q für Zahlen von 1 bis 10 steht.

Eine weitere bevorzugte Gruppe von Penetrationsförderern sind Alkanol-alkoxylate der Formel

$$R\text{-}O\text{-}(\text{-}BO\text{-})_r\text{-}(\text{-}EO\text{-})_S\text{-}R' \qquad (III'\text{-}e)$$

in welcher

R und R' die oben angegebenen Bedeutungen haben,

BO für

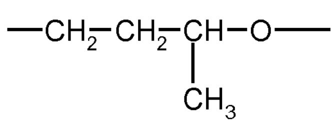

steht,

EO für $CH_2$-$CH_2$-O- steht,

r für Zahlen von 1 bis 10 steht und

s für Zahlen von 1 bis 10 steht.

Eine weitere bevorzugte Gruppe von Penetrationsförderern sind Alkanol-Alkoxylate der Formel

$$CH_3\text{-}(CH_2)_t\text{-}CH_2\text{-}O\text{-}(\text{-}CH_2\text{-}CH_2\text{-}O\text{-})_u\text{-}R' \qquad (III'\text{-}f)$$

in welcher

R' die oben angegebene Bedeutung hat,

t für Zahlen von 8 bis 13 steht

u für Zahlen von 6 bis 17 steht.

In den zuvor angegebenen Formeln steht

R vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (III-c) sei 2-Ethyl-hexyl-alkoxylat der Formel

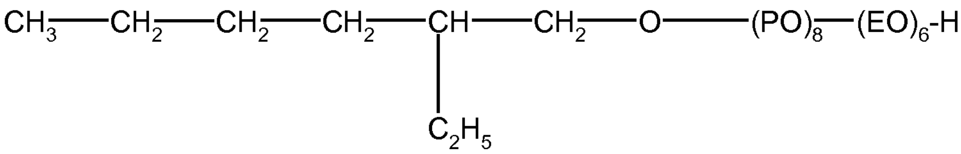

(III'-c-1)

in welcher

EO für $-CH_2-CH_2-O-$ steht,
PO für

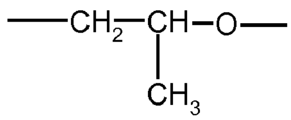

steht und die Zahlen 8 und 6 Durchschnittswerte darstellen, genannt.

Als Beispiel für ein Alkanol-Alkoxylat der Formel (III-d) sei die Formel

$$CH_3-(CH_2)_{10}-O-(-EO-)_6-(-BO-)_2-CH_3 \qquad (III'\text{-d-1})$$

in welcher

EO für $CH_2-CH_2-O-$ steht,
BO für

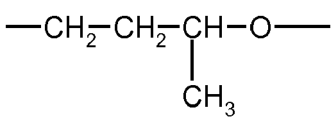

steht und

die Zahlen 10, 6 und 2 Durchschnittswerte darstellen, genannt.

Besonders bevorzugte Alkanol-Alkoxylate der Formel (III'-f) sind Verbindungen dieser Formel, in denen

t für Zahlen von 9 bis 12 und
u für Zahlen von 7 bis 9

steht.

Ganz besonders bevorzugt genannt sei Alkanol-Alkoxylat der Formel (III'-f-1)

$$CH_3-(CH_2)_t-CH_2-O-(-CH_2-CH_2-O-)_u-H \qquad (III'\text{-f-1})$$

in welcher

t für den Durchschnittswert 10,5 steht und

u für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98-35 553, WO 00-35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sojabohnenöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäß hervorgehobene Kombinationen von Wirkstoff, Salz und Penetrations-förderer sind in folgender Tabelle aufgeführt. "gemäß Test" bedeutet dabei, dass jede Verbindung geeignet ist, die in dem Test für die Kutikelpenetration (Baur et al., 1997, *Pesticide Science* **51**, 131-152) als Penetrationsförderer wirkt.

| # | Wirkstoff | Salz | Penetrationsförderer |
|---|---|---|---|
| 1 | A.1 | Ammoniumsulfat | gemäß Test |
| 2 | A.1 | Ammoniumlaktat | gemäß Test |
| 3 | A.1 | Ammoniumnitrat | gemäß Test |
| 4 | A.1 | Ammoniumthiosulfat | gemäß Test |
| 5 | A.1 | Ammoniumthiocyanat | gemäß Test |
| 6 | A.1 | Ammoniumcitrat | gemäß Test |
| 7 | A.1 | Ammoniumoxalat | gemäß Test |
| 8 | A.1 | Ammoniumformiat | gemäß Test |
| 9 | A.1 | Ammoniumhydrogenphosphat | gemäß Test |
| 10 | A.1 | Ammoniumdihydrogenphosphat | gemäß Test |
| 11 | A.1 | Ammoniumcarbonat | gemäß Test |
| 12 | A.1 | Ammoniumbenzoat | gemäß Test |
| 13 | A.1 | Ammoniumsulfit | gemäß Test |
| 14 | A.1 | Ammoniumbenzoat | gemäß Test |
| 15 | A.1 | Ammoniumhydrogenoxalat | gemäß Test |
| 16 | A.2 | Ammoniumsulfat | gemäß Test |
| 17 | A.2 | Ammoniumlaktat | gemäß Test |
| 18 | A.2 | Ammoniumnitrat | gemäß Test |
| 19 | A.2 | Ammoniumthiosulfat | gemäß Test |
| 20 | A.2 | Ammoniumthiocyanat | gemäß Test |
| 21 | A.2 | Ammoniumcitrat | gemäß Test |
| 22 | A.2 | Ammoniumoxalat | gemäß Test |
| 23 | A.2 | Ammoniumformiat | gemäß Test |
| 24 | A.2 | Ammoniumhydrogenphosphat | gemäß Test |

(fortgesetzt)

| # | Wirkstoff | Salz | Penetrationsförderer |
|---|---|---|---|
| 25 | A.2 | Ammoniumdihydrogenphosphat | gemäß Test |
| 26 | A.2 | Ammoniumcarbonat | gemäß Test |
| 27 | A.2 | Ammoniumbenzoat | gemäß Test |
| 28 | A.2 | Ammoniumsulfit | gemäß Test |
| 29 | A.2 | Ammoniumbenzoat | gemäß Test |
| 30 | A.2 | Ammoniumhydrogenoxalat | gemäß Test |
| 31 | A.3 | Ammoniumsulfat | gemäß Test |
| 32 | A.3 | Ammoniumlaktat | gemäß Test |
| 33 | A.3 | Ammoniumnitrat | gemäß Test |
| 34 | A.3 | Ammoniumthiosulfat | gemäß Test |
| 35 | A.3 | Ammoniumthiocyanat | gemäß Test |
| 36 | A.3 | Ammoniumcitrat | gemäß Test |
| 37 | A.3 | Ammoniumoxalat | gemäß Test |
| 38 | A.3 | Ammoniumformiat | gemäß Test |
| 39 | A.3 | Ammoniumhydrogenphosphat | gemäß Test |
| 40 | A.3 | Ammoniumdihydrogenphosphat | gemäß Test |
| 41 | A.3 | Ammoniumcarbonat | gemäß Test |
| 42 | A.3 | Ammoniumbenzoat | gemäß Test |
| 43 | A.3 | Ammoniumsulfit | gemäß Test |
| 44 | A.3 | Ammoniumbenzoat | gemäß Test |
| 45 | A.3 | Ammoniumhydrogenoxalat | gemäß Test |
| 46 | A.4 | Ammoniumsulfat | gemäß Test |
| 47 | A.4 | Ammoniumlaktat | gemäß Test |
| 48 | A.4 | Ammoniumnitrat | gemäß Test |
| 49 | A.4 | Ammoniumthiosulfat | gemäß Test |
| 50 | A.4 | Ammoniumthiocyanat | gemäß Test |
| 51 | A.4 | Ammoniumcitrat | gemäß Test |
| 52 | A.4 | Ammoniumoxalat | gemäß Test |
| 53 | A.4 | Ammoniumformiat | gemäß Test |
| 54 | A.4 | Ammoniumhydrogenphosphat | gemäß Test |
| 55 | A.4 | Ammoniumdihydrogenphosphat | gemäß Test |
| 56 | A.4 | Ammoniumcarbonat | gemäß Test |
| 57 | A.4 | Ammoniumbenzoat | gemäß Test |
| 58 | A.4 | Ammoniumsulfit | gemäß Test |
| 59 | A.4 | Ammoniumbenzoat | gemäß Test |
| 60 | A.4 | Ammoniumhydrogenoxalat | gemäß Test |
| 61 | A.5 | Ammoniumsulfat | gemäß Test |
| 62 | A.5 | Ammoniumlaktat | gemäß Test |

(fortgesetzt)

| # | Wirkstoff | Salz | Penetrationsförderer |
|---|---|---|---|
| 63 | A.5 | Ammoniumnitrat | gemäß Test |
| 64 | A.5 | Ammoniumthiosulfat | gemäß Test |
| 65 | A.5 | Ammoniumthiocyanat | gemäß Test |
| 66 | A.5 | Ammoniumcitrat | gemäß Test |
| 67 | A.5 | Ammoniumoxalat | gemäß Test |
| 68 | A.5 | Ammoniumformiat | gemäß Test |
| 69 | A.5 | Ammoniumhydrogenphosphat | gemäß Test |
| 70 | A.5 | Ammoniumdihydrogenphosphat | gemäß Test |
| 71 | A.5 | Ammoniumcarbonat | gemäß Test |
| 72 | A.5 | Ammoniumbenzoat | gemäß Test |
| 73 | A.5 | Ammoniumsulfit | gemäß Test |
| 74 | A.5 | Ammoniumbenzoat | gemäß Test |
| 75 | A.5 | Ammoniumhydrogenoxalat | gemäß Test |
| 76 | A.6 | Ammoniumsulfat | gemäß Test |
| 77 | A.6 | Ammoniumlaktat | gemäß Test |
| 78 | A.6 | Ammoniumnitrat | gemäß Test |
| 79 | A.6 | Ammoniumthiosulfat | gemäß Test |
| 80 | A.6 | Ammoniumthiocyanat | gemäß Test |
| 81 | A.6 | Ammoniumcitrat | gemäß Test |
| 82 | A.6 | Ammoniumoxalat | gemäß Test |
| 83 | A.6 | Ammoniumformiat | gemäß Test |
| 84 | A.6 | Ammoniumhydrogenphosphat | gemäß Test |
| 85 | A.6 | Ammoniumdihydrogenphosphat | gemäß Test |
| 86 | A.6 | Ammoniumcarbonat | gemäß Test |
| 87 | A.6 | Ammoniumbenzoat | gemäß Test |
| 88 | A.6 | Ammoniumsulfit | gemäß Test |
| 89 | A.6 | Ammoniumbenzoat | gemäß Test |
| 90 | A.6 | Ammoniumhydrogenoxalat | gemäß Test |
| 91 | A.7 | Ammoniumsulfat | gemäß Test |
| 92 | A.7 | Ammoniumlaktat | gemäß Test |
| 93 | A.7 | Ammoniumnitrat | gemäß Test |
| 94 | A.7 | Ammoniumthiosulfat | gemäß Test |
| 95 | A.7 | Ammoniumthiocyanat | gemäß Test |
| 96 | A.7 | Ammoniumcitrat | gemäß Test |
| 97 | A.7 | Ammoniumoxalat | gemäß Test |
| 98 | A.7 | Ammoniumformiat | gemäß Test |
| 99 | A.7 | Ammoniumhydrogenphosphat | gemäß Test |
| 100 | A.7 | Ammoniumdihydrogenphosphat | gemäß Test |

(fortgesetzt)

| # | Wirkstoff | Salz | Penetrationsförderer |
|---|---|---|---|
| 101 | A.7 | Ammoniumcarbonat | gemäß Test |
| 102 | A.7 | Ammoniumbenzoat | gemäß Test |
| 103 | A.7 | Ammoniumsulfit | gemäß Test |
| 104 | A.7 | Ammoniumbenzoat | gemäß Test |
| 105 | A.7 | Ammoniumhydrogenoxalat | gemäß Test |
| 106 | A.8 | Ammoniumsulfat | gemäß Test |
| 107 | A.8 | Ammoniumlaktat | gemäß Test |
| 108 | A.8 | Ammoniumnitrat | gemäß Test |
| 109 | A.8 | Ammoniumthiosulfat | gemäß Test |
| 110 | A.8 | Ammoniumthiocyanat | gemäß Test |
| 111 | A.8 | Ammoniumcitrat | gemäß Test |
| 112 | A.8 | Ammoniumoxalat | gemäß Test |
| 113 | A.8 | Ammoniumformiat | gemäß Test |
| 114 | A.8 | Ammoniumhydrogenphosphat | gemäß Test |
| 115 | A.8 | Ammoniumdihydrogenphosphat | gemäß Test |
| 116 | A.8 | Ammoniumcarbonat | gemäß Test |
| 117 | A.8 | Ammoniumbenzoat | gemäß Test |
| 118 | A.8 | Ammoniumsulfit | gemäß Test |
| 119 | A.8 | Ammoniumbenzoat | gemäß Test |
| 120 | A.8 | Ammoniumhydrogenoxalat | gemäß Test |

Als Lösemittel in den erfindungsgemäßen Zusammensetzungen lassen sich alle üblicherweise in agrochemischen Formulierungen genannten, wassermischbaren Lösemittel einsetzen, in denen die Wirkstoffe der Formeln (I) und (I') in den hier verwendeten Konzentrationen lösen. Als Beispiele genannt seien Wasser, Alkohole wie Methanol, Ethanol oder Isopropanol, Ether bzw. Polyether wie 1,4-Dioxan, Tetrahydrofuran oder Dimethoxyethan, Amide wie Formamid, Acetamid, N,N-Dimethylformamid , N,N-Dimethylacetamid oder Hallcomid® (Mischung aus 50 - 60 % N,N-Dimethyloctanamid und 35 - 45 % N,N-Dimethyldecanamid), Sulfoxide/Sulfone wie Dimethylsulfoxid oder Sulfolan und Laktone/Laktame wie N-Methylpyrrolidon und gamma-Butyrolakton.

Bevorzugt als Lösemittel ist Wasser.

Für die Herstellung der erfindungsgemäßen wasserlöslichen Konzentrate enthaltend eine Verbindung der Formel (I) ist es vorteilhaft, die Verbindung der Formel (I) in-situ während der Herstellung der Zusammensetzung durch Umsetzen der entsprechenden Verbindung der Formel (I') mit einer geigneten Base zu erzeugen.

Dieses Verfahren ermöglicht die Herstellung von Zusammensetzungen enhaltend Verbindungen der Formel (I), die in isolierter Form teilweise wenig stabil sind. Als Basen geeignet sind prinzipiell alle organischen und anorganischen Basen, soweit deren Verwendung für landwirtschaftliche Zwecke unbedenklich ist.

Beispiele für Basen sind

a) Metallhydroxyde wie beispielsweise Lithium-, Natrium- und Kaliumhydroxyd, Magnesium- und Calciumhydroxid, Aluminiumhydroxid, Zinkhydroxid oder Kupferhydroxyd,

b) Metalloxide wie beispielsweise Lithium-, Natrium- und Kaliumoxid oder Aluminiumoxid

c) Amine der allgemeinen Formel $NR^1R^2R^3$. Darin können $R^1$, $R^2$ und $R^3$ gleich oder verschieden sein und stehen jeweils für Wasserstoff, $C_1$-$C_5$-Allcyl, $C_1$-$C_5$-Isoallcyl oder $C_3$-$C_7$-Cycloalkyl, die wiederum jeweils ein- oder mehrfach mit Fluor, Chlor, Brom, Cyano, Hydroxy substituiert oder durch ein- oder mehrere Sauerstoff- oder Schwefelatome

unterbrochen sein können.
Spezielle Beispiele sind Ammoniak, Methylamin, Dimethylamin, Triethylamin, Ethylamin, Diethylamin, Triethylamin, Isopropylamin, Ethanolamin, Diethanolamin, Triethanolamin, 2-Diethylaminoethanol, Diisopropylamin, Cyclohexylamin, Dicyclohexylamin,

d) Mono-, bi- oder tricyclische Amine wie beispielsweise Morpholin, Thiomorpholin, Piperidin, Pyrrolidin, 1,4-Diazabicyclo[2.2.2]octane (DABCO) oder 1,5-Diazabicyclo-[4.3.0]un-dec-7-en (DBU),

e) Diamine wie beispielsweise N,N-Bis-(2-hydroxyethyl)-$C_8$-$C_{18}$-alkylamine, Hexamethylentetramin, N,N,N',N'-Tetrakis-(2-hydroxypropyl)-ethylendiamin, 2-Diethylamino-ethylamin, N,N,N',N'-Tetraethyl-ethylendiamin, N,N,N',N'-Tetramethyl-ethylendiamin, 2-(2-Aminoethylamino)-ethanol oder Lysin,

f) Aromatische Amine wie beispielsweise Pyridin, 2-Methyl-pyridin, 3-Methylpyridin, 4-Methylpyridin, 2,4-Dimethylpyridin, 2,5-Dimethyl-pyridin, 2,6-Dimethylpyridin, 5-Ethyl-2-methylpyridin, Pyrrol, Imidazol, Chinolin, Chinoxalin, 1,2-Dimethylimidazol, 1,3-Dimethylimidazolium-methylsulfat,

g) Carbonate, wie beispielsweise Kaliumcarbonat, Kaliumhydrogencarbonat, Natriumcarbonat, Natriumhydrogencarbonat, Magnesiumcarbonat, Calciumcarbonat, Kupfercarbonat, Zinkcarbonat oder Lithiumcarbonat,

h) Sulfite, wie beispielsweise Natrium-, Kalium-, Lithium-, oder Zinksulfit,

i) Phosphate wie beispielsweise Lithium- Kalium-, Natrium-, Calcium- und Magnesiumphosphat, Lithium-, Kalium-Natrium-, Calcium- und Magnesiumhydrogenphosphat oder Kalium- und Natriumdihydrogenphosphat,

j) Alkoholate wie beispielsweise Lithium, Natrium- und Kaliummethylat oder -ethylat,

k) Ammoniumhydroxyde, wie beispielsweise Trimethyl-, Triethyl- Tripropyl- oder Tributylammoniumhydroxyd, Tetramethylammoniumhydroxid, Tetraethylammoniumhydroxid, Tetraethanolammoniumhydroxid oder Methyltriethylammoniumhydroxid,

l) Amidine und Guanidine, die jeweils gegebenenfalls substituiert sein können, beispielsweise Acetamidin, Formamidin, Guanidin, 1,1,3,3-Tetraaminoguaniden, Aminoguanidin oder Arginin,

m) Basische Carbonsäuresalze, bevorzugterweise Acetate wie beispielsweise Lithium-, Natrium- oder Kaliumacetat, Oxalate wie beispielsweise Natrium- oder Kaliumoxalat, Tartrate wie Natrium- oder Kaliumtartrat sowie Citrate, wie beispielsweise Natrium- oder Kaliumcitrat,

n) mit Hydroxidionen beladene stark oder schwach basische Anionenaustauscher, beispielsweise solche, die unter den Namen AMBERLITE®, AMBERLYST®, DUOLITE®, DOWEX® oder LEWATITE® kommerziell erhältlich sind,

o) basische Ammoniumsalze (wie z. B. Diammonium-hydrogenphosphat).

Die Base kann grundsätzlich auch in immobilisierter Form eingesetzt werden, wobei das Trägermaterial nach Herstellung des erfindungsgemäßen Konzentrates abgetrennt werden kann, beispielsweise durch Filtration.
Je nach Notwendigkeit können - bezogen auf die Menge der eingesetzten Verbindungen der allgemeinen Formel (I') - zwischen 0,1 und 100 Moläquivalente, typischerweise 0,5 bis 3 Moläquivalente, der Base verwendet werden.
Bevorzugt als basische Hilfsstoffe sind Lithiumhydroxid, Natriumhydroxid, Kaliumhydroxid, Magnesiumhydroxyd, Calciumhydroxid sowie Ammoniak, basische Ammoniumsalze (wie z. B. Diammonium-hydrogenphosphat), Alkylamine und Hydroxyalkylamine.
Optional enthalten die erfindungsgemäßen Formulierungen weitere Zusatzstoffe aus den Gruppen der schaumhemmenden Mittel, der Antioxydantien und/oder der Farbstoffe.
Als schaumhemmende Stoffe kommen alle üblicherweise für diesen Zweck in agrochemischen Mitteln einsetzbaren Substanzen in Betracht. Bevorzugt sind Polydimethylsiloxane, Silikonöle und Magnesiumstearat.
Als Antioxydantien kommen alle üblicherweise für diesen Zweck in agrochemischen Mitteln einsetzbaren Substanzen in Betracht. Bevorzugt ist Butylhydroxytoluol (2,6-di-t-butyl-4-methylphenol, BHT).
Als Farbstoffe kommen alle üblicherweise für diesen Zweck in agrochemischen Mitteln einsetzbaren Substanzen in Frage. Beispielhaft genannt seien Titandioxid, Farbruß, Zinkoxid und Blaupigmente sowie Permanentrot FGR.
In den erfindungsgemäßen Formulierungen beträgt der Gehalt an Wirkstoff im Allgemeinen 0,1 bis 50 Gew.-

%, bevorzugt 1 bis 25 Gew.-%, besonders bevorzugt 2 bis 20 Gew.-%.

In den erfindungsgemäßen Formulierungen beträgt der Gehalt an Tensid (Wirksubstanz, evtl. korrigiert um Wassergehalt) im Allgemeinen 5 bis 50 Gew.-% und bevorzugt 10 bis 30 Gew.-%.

In anwendungsfertigen Formulierungen (Spritzbrühen) beträgt der Gehalt an Tensid im Allgemeinen 0,1 bis 10 g/l, bevorzugt 0,3 bis 3 g/l.

Das Tensid wird im Allgemeinen in einer Aufwandmenge von 20 bis 1000 g a.i. / ha, bevorzugt 100 bis 300 g a.i. / ha ausgebracht.

Verwendet man beispielsweise gemäß Verfahren (A) N-(2-6-Dimethyl-4-chlor-4,4-ethylendioxyphenylacetyl)-1-amino-cyclohexancarbonsäure-ethylester als Ausgangsstoff, so kann der Verlauf des erfindungsgemäßen Verfahrens durch folgendes Reaktionsschema wiedergegeben werden:

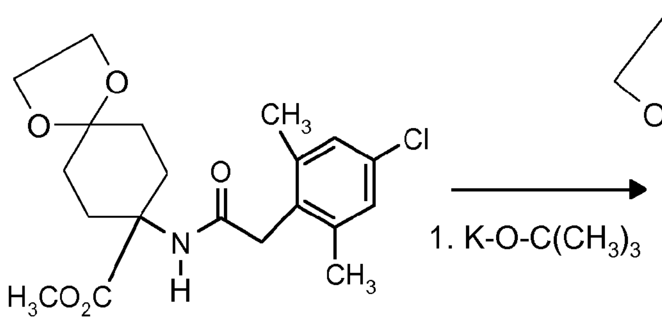


Verwendet man beispielsweise gemäß Verfahren (B) 8,8'-Ethylendioxy-3-[(2,6-dimethyl-4-chlor)-phenyl]-1-azaspiro[4,5]-decan-2,4-dion und NaOH als Komponenten, so kann der Verlauf des erfindungsgemäßen Verfahrens durch folgendes Reaktionsschema wiedergegeben werden:

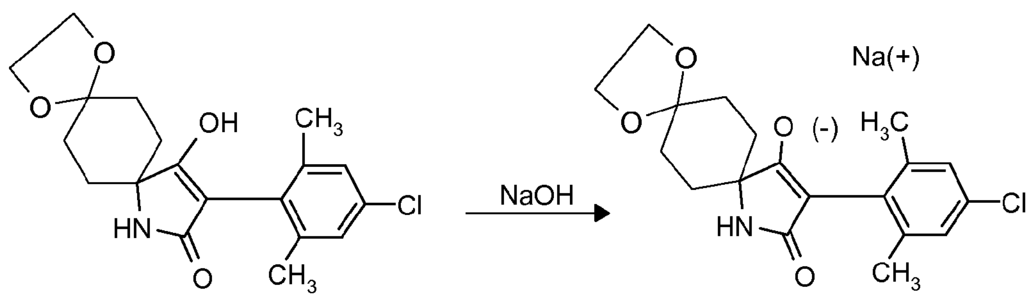


Die beim erfindungsgemäßen Verfahren (A) als Ausgangsstoffe benötigten Verbindungen der Formel (II)

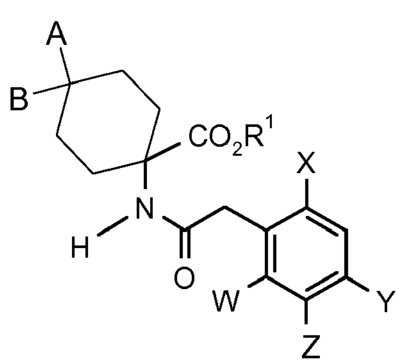

(II)

in welcher
A, B, W, X, Y, Z und $R^1$ die oben angegebenen Bedeutungen haben,
sind teilweise bekannt aus WO 06/089633 oder lassen sich nach den dort beschriebenen Verfahren herstellen.

Die zur Durchführung des erfindungsgemäßen Verfahren (B-$\alpha$) außerdem als Ausgangsstoffe benötigten Metallhydroxide, Metallalkoxide oder Metallhydride der Formeln (III) oder (IV) sind allgemein bekannte Verbindungen der Anorganischen Chemie.

Die zur Durchführung des erfindungsgemäßen Verfahren (B-$\beta$) außerdem als Ausgangsstoffe benötigten Amine der Formel (V) oder Ammoniumverbindungen der Formel (VI) sind allgemein bekannte Verbindungen der organischen Chemie.

Die Verbindungen der Formeln (I') und (II) sind darüber hinaus aus den eingangs zitierten Patentanmeldungen bekannt und/oder lassen sich nach den dort angegebenen Methoden herstellen.

Das Verfahren (A) ist **dadurch gekennzeichnet, dass** man Verbindungen der Formel (II), in welcher A, B, W, X, Y, Z und $R^1$ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, IsoPropanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl($C_8$-$C_{10}$)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (B) ist **dadurch gekennzeichnet, dass** man Verbindungen der Formel (I') mit Metallhydroxiden bzw. Metallalkoxiden der Formel (III) oder Metallhydriden der Formel (IV), gegebenenfalls in Gegenwart eines Verdünnungsmittels, umsetzt.

Als Verdünnungsmittel können bei dem erfindungsgemäßen Verfahren (B) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (B) wird im Allgemeinen unter Normaldruck durchgeführt.

Die Reaktionstemperaturen liegen im Allgemeinen zwischen -20°C und 100°C, vorzugsweise zwischen 0°C und 50°C.

Die erfindungsgemäßen Wirkstoffe/Wirkstoffkombinationen eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora,

Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp.,

Sogatella furcifera, Sogatodes spp., Stictocephala festina, Tenalaphara malayensis, Tinocallis caryaefoliae,

Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Aphelenchoides spp., Bursaphelenchus spp.,

Ditylenchus dipsaci, Globodera spp., Heterodera spp., Longidorus spp., Meloidogyne spp., Pratylenchus spp., Radopholus similis, Trichodorus spp., Tylenchulus semipenetrans, Xiphinema spp.

Die erfindungsgemäßen Verbindungen/Wirkstoffkombinationen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen/Wirkstoff kombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Samen, weiterhin durch ein- oder mehrschichtiges Umhüllen.

Die Wirkstoffe/Wirkstoffkombinationen können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe/Wirkstoffkombinationen mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie

Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gew.-% Wirkstoff, vorzugsweise zwischen 0,5 und 90 %.

Der erfindungsgemäße Wirkstoff/Wirkstoffkombinationen kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe/Wirkstoffkombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muss.

Die erfindungsgemäßen Wirkstoffe/Wirkstoffkombinationen können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepassten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen

bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe/Wirkstoffkombinationen wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis

spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe/Wirkstoffkombinationen der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe/Wirkstoffkombinationen geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen/Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im Allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise $\alpha$-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher (gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden

Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

Algizide wie

2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

Fungizide wie

Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie

Azaconazole, Cyproconazole, Epoxyconazole, Hexaconazole, Metconazole, Propiconazole und Tebuconazole;

Molluskizide wie

Fentinacetat, Metaldehyd, Methiocarb, Niclosamid, Thiodicarb und Trimethacarb, Fe-chelate;

oder herkömmliche Antifouling-Wirkstoffe wie

4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridintriphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer,

*Chem. Ind.* 1985, *37*, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis,

Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe/Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe/Wirkstoffkombinationen können z.B. bei den folgenden Pflanzen verwendet werden:

<u>Dikotyle Unkräuter der Gattungen:</u> Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

<u>Dikotyle Kulturen der Gattungen:</u> Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

<u>Monokotyle Unkräuter der Gattungen:</u> Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

<u>Monokotyle Kulturen der Gattungen:</u> Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe/Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe/Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I)/Wirkstoffkombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe/Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe/Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B.

gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe/Wirkstoffkombinationen können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe/Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe/Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im Allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im Allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfähren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im Allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Die Herstellung und Verwendung der erfindungsgemäßen Wirkstoffe/Wirkstoffkombinationen geht aus den

nachfolgenden Beispielen hervor.

**Herstellungsbeispiele**

Beispiel A.1

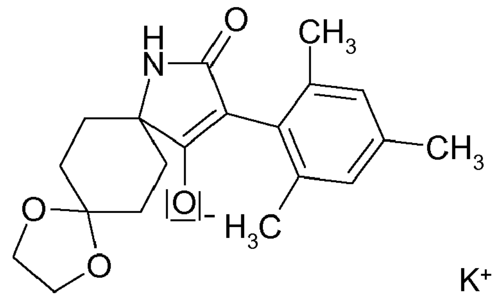

0,343 g (1 mmol) der Verbindung (I'-1) werden in eine Lösung aus 10 ml Wasser und 1 ml 1 N Kalilauge bei Raumtemperatur portionsweise eingetragen. Man rührt 1 h nach, rotiert im Vakuum ein und fällt den Rückstand aus Methyl-tert.-butylether/n-Hexan und saugt ab.

Ausbeute: 0.36 g (= 94 % d. Theorie)
Ionenchromatographie: $K^+$ berechnet 10.2 %
$K^+$ gefunden 10.15%
$^1$H-NMR(400 MHz, $d_6$-DMSO): $\delta$ = 1.23, 1.26 (dm, 2H), 1.65-1.76 (m, 4H), 1.90-1.98 (dt, 2H), 2.07 (s, 6H, Ar-$\underline{CH_3}$), 2.16 (s, 3H, Ar-4-$\underline{CH_3}$), 3.85 (s, 4H, O-$\underline{CH_2}$-$\underline{CH_2}$-O), 6.63 (s, 2H, Ar-$\underline{H}$)

In Analogie zu Beispiel A.1 und gemäß den allgemeinen Angaben zur Herstellung von Verbindungen der Formel (I) erhält man folgende Beispiele:

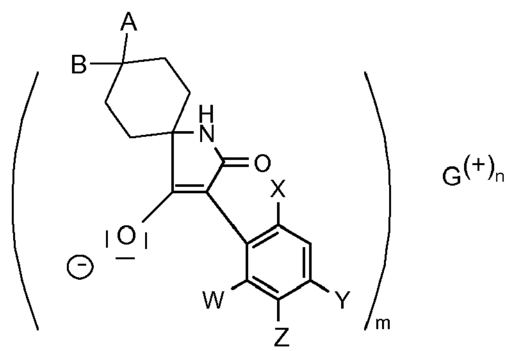

(I)

| Bsp.-Nr. | W | X | Y | Z | A | B | $G^{(+)n}$ | m | $^1$H-NMR (400 MHz, $d_6$-DMSO) |
|---|---|---|---|---|---|---|---|---|---|
| A.2 | $CH_3$ | $CH_3$ | $CH_3$ | H | -O-$(CH_2)_2$-O- | | $Na^+$ | 1 | 1.24, 1.27 (dm, 2H), 1.64-1.77 (m, 4H), 1.91-1.99 (dt,2H), 2.07 (s,6H, Ar-$\underline{CH_3}$), 2.16 (s,3H,Ar-4-$\underline{CH_3}$), 3.85 (s,4H,-O-($\underline{CH_2}$)$_2$-O), 6.64 (s, 2H, Ar-$\underline{H}$) |

(fortgesetzt)

| Bsp.-Nr. | W | X | Y | Z | A | B | $G^{(+)n}$ | m | $^1$H-NMR (400 MHz, $d_6$-DMSO) |
|---|---|---|---|---|---|---|---|---|---|
| A.3 | $CH_3$ | $CH_3$ | $CH_3$ | H | $-O-(CH_2)_2-O-$ | | $Li^+$ | 1 | 1.22, 1.26 (dm, 2H), 1.65-1.75 (m, 4H), 1.90-1.98 (dt, 2H), 2.07 (s, 6H, Ar-$CH_3$), 2.16 (s, 3H, Ar-4-$CH_3$), 3.85 (s, 4H, -O-$(CH_2)_2$-O), 6.63 (s, 2H, Ar-H) |
| A.4 | $CH_3$ | $CH_3$ | Cl | H | $-O-(CH_2)_2-O-$ | | $Li^+$ | 1 | 1.24, 1.26 (dm,2H), 1.65-1.77 (m, 4H), 1.91-1.99 (dt, 2H), 2.12 (s, 6H, Ar-$CH_3$), 3.85 (s,4H,O-$(CH_2)_2$-O), 6.85 (s,2H,Ar-H) |
| A.5 | $CH_3$ | $CH_3$ | Cl | H | $-O-(CH_2)_2-O-$ | | $Na^+$ | 1 | 1.24, 1.27 (dm,2H), 1.65-1.77(m, 4H), 1.91-1.98 (dt,2H), 2.12 (s,6H, Ar-$CH_3$), 3.85 (s,4H,O-$(CH_2)_2$-O), 6.86 (s,2H,Ar-H) |
| A.6 | $CH_3$ | $CH_3$ | Cl | H | $-O-(CH_2)_2-O-$ | | $K^+$ | 1 | 1.23, 1.26 (dm,2H), 1.64-1.76(m, 4H), 1.89-1.97 (dt,2H), 2.12(s,6H, Ar-$CH_3$), 3.85 (s,4H, O-$(CH_2)_2$-O), 6.84 (s,2H,Ar-H) |
| A.7 | $CH_3$ | $CH_3$ | Cl | H | $-O-(CH_2)_2-O-$ | | $Mg^{2+}$ | 2 | 1.28, 1.31 (dm,2H), 1.69-1.72 (m, 4H), 1.93-1.99 (m,2H), 2.12 (s,6H, Ar-$CH_3$), 3.86 (s,4H,O-$(CH_2)_2$-O, 6.89 (s,2H,Ar-H) |
| A.8 | $CH_3$ | $CH_3$ | Cl | H | $-O-(CH_2)_2-O-$ | | $Ca^{2+}$ | 2 | 1.25, 1.28 (dm,2H), 1.66-1.78 (m, 4H), 1.95-1.99 (dt, 2H), 2.13 (s,6H, Ar-$CH_3$), 3.86 (s,4H,O-$(CH_3)_2$-O), 6.87 (s,2H, Ar-H) |

Herstellung von Suspensionskonzentraten (SC-Formulierungen)

Der Wirkstoff wurde in eine wässrige Lösung eines geeigneten Tensids eingerührt. Die Dispersion wurde fein gemahlen unter Benutzung von Standard-Vorrichtungen bis eine durchschnittliche Teilchengröße von weniger als 5 μm erreicht war. Eine typische Zusammensetzung einer SC025-Formulierung war:

| | |
|---|---|
| A.5 | 25 g/l |
| Soprophor TS 29 | 40 g/l |
| Glycerin | 100 g/l |
| Zitronensäure | 1 g/l |
| Silikonentschäumer | 1 g/l |
| Proxel GXL 20 % | 1.2 g/l |
| Preventol D 7 | 8 g/l |
| Wasser | 224 g/l |
| Kelzan SMX 2 | 600 g/l |

Herstellung von wasserlöslichen Konzentrationen (SL-Formulierungen)

Zur Herstellung der SL-Formulierung werden Wasser, der Wirkstoff (I'-2) und Harnstoff vorgelegt. Man gibt 2 molare Natronlauge zu bis alles in Lösung geht und stellt anschließend mit 1 molarer Salzsäure auf pH10 ein und füllt mit Wasser auf 1 1 auf.

Eine typische Zusammensetzung einer SL-Formulierung war

| | |
|---|---|
| A.5 | 50 g |

(fortgesetzt)

| | |
|---|---|
| Natronlauge (2 m) | 51.35 g |
| Salzsäure (1 m) | 51.35 g |
| Harnstoff | 102.7 g |
| Preventol D 7 | 0.82 g |
| Proxel GXL 20 % | 1.23 g |
| Silfoam SRE | 1.03 g |
| Wasser | 768.5 g |

| Spritzbrühenverhalten von A.5 SL 050 und SC 050 bei verschieden pH-Werten | | | | | | | | | | |
|---|---|---|---|---|---|---|---|---|---|---|
| Nr. | Formulier-typ | Lösung | pH-Wert der Lösung | soll a.i. in 300 L $H_2O$ [g] | Einwaage Formulierung [g] | Beurteilung des a.i. Verhaltens in der Spritzbrühen nach | | | | |
| | | | | | | 0 min | 5 min | 30 min | 1 h | 24 h |
| 1 | SL 050 | 500 ppm $H_2O$ | 6,8 | 12 | 0,08 | gelöst | gelöst | gelöst | gelöst | gelöst |
| 2 | | Puffer | 6,0 | 12 | 0,08 | gelöst | gelöst | gelöst | gelöst | gelöst |
| 3 | | Puffer | 8,0 | 12 | 0,08 | gelöst | gelöst | gelöst | gelöst | gelöst |
| 4 | SC050 | 500 ppm $H_2O$ | 5,3 | 12 | 0,08 | **nicht gelöst** | **nicht gelöst** | **nicht gelöst** | **nicht gelöst** | **nicht gelöst** |
| 5 | | Puffer | 6,0 | 12 | 0,08 | **nicht gelöst** | **nicht gelöst** | **nicht gelöst** | **nicht gelöst** | **nicht gelöst** |
| 6 | | Puffer | 8,0 | 12 | 0,08 | **nicht gelöst** | gelöst | gelöst | gelöst | gelöst |
| 7 | SL 050 | 500 ppm $H_2O$ | 6,6 | 6 | 0,04 | gelöst | gelöst | gelöst | gelöst | gelöst |
| 8 | | Puffer | 6,0 | 6 | 0,04 | gelöst | gelöst | gelöst | gelöst | gelöst |
| 9 | | Puffer | 8,0 | 6 | 0,04 | gelöst | gelöst | gelöst | gelöst | gelöst |
| 10 | SC050 | 500 ppm $H_2O$ | 5,4 | 6 | 0,04 | **nicht gelöst** | **nicht gelöst** | **nicht gelöst** | **nicht gelöst** | **nicht gelöst** |
| 11 | | Puffer | 6,0 | 6 | 0,04 | **nicht gelöst** | **nicht gelöst** | **fast gelöst** | **fast gelöst** | **fast gelöst** |
| 12 | | Puffer | 8,0 | 6 | 0,04 | **nicht gelöst** | gelöst | gelöst | gelöst | gelöst |

## Anwendungsbeispiele

### Beispiel A

Steigerung der Penetration in die Pflanze durch Ammonium- oder Phosphoniumsalze und synergistische Steigerung der Penetration in die Pflanze durch Ammonium- / Phosphoniumsalze in Kombination mit Penetrationsförderern

In diesem Test wurde die Penetration von Wirkstoffen durch enzymatisch isolierte Kutikeln von Apfelbaumblättern gemessen.

Verwendet wurden Blätter, die in voll entwickeltem Zustand von Apfelbäumen der Sorte Golden Delicious abgeschnitten wurden. Die Isolierung der Kutikeln erfolgte in der Weise, dass

- zunächst auf der Unterseite mit Farbstoff markierte und ausgestanzte Blattscheiben mittels Vakuuminfiltration mit einer auf einen pH Wert zwischen 3 und 4 gepufferten Pectinase-Lösung (0,2 bis 2 %ig) gefüllt wurden,
- dann Natriumazid hinzugefügt wurde und
- die so behandelten Blattscheiben bis zur Auflösung der ursprünglichen Blattstruktur und zur Ablösung der nicht zellulären Kutikula stehen gelassen wurden.

Danach wurden nur die von Spaltöffnungen und Haaren freien Kutikeln der Blattoberseiten weiter verwendet. Sie wurden mehrfach abwechselnd mit Wasser und einer Pufferlösung vom pH Wert 7 gewaschen. Die erhaltenen sauberen Kutikel wurden schließlich auf Teflonplättchen aufgezogen und mit einem schwachen Luftstrahl geglättet und getrocknet.

Im nächsten Schritt wurden die so gewonnenen Kutikelmembranen für Membran-Transport-Untersuchungen in Diffusionszellen (= Transportkammern) aus Edelstahl eingelegt. Dazu wurden die Kutikeln mit einer Pinzette mittig auf die mit Silikonfett bestrichenen Ränder der Diffusionszellen plaziert und mit einem ebenfalls gefetteten Ring verschlossen. Die Anordnung war so gewählt worden, dass die morphologische Außenseite der Kutikeln nach außen, also zur Luft, gerichtet war, während die ursprüngliche Innenseite dem Inneren der Diffusionszelle zugewandt war.

Die Diffusionszellen waren mit einer 30%igen Ethylenglykol/Wasser-Lösung befüllt. Zur Bestimmung der Penetration wurden jeweils 10 $\mu$l der Spritzbrühe der nachstehenden Zusammensetzung auf die Außenseite der Kutikula appliziert. Das Ansetzen der Spritzbrühe erfolgt mit lokalem Leitungswasser mittlerer Wasserhärte.

Nach dem Auftragen der Spritzbrühen ließ man das Wasser verdunsten, drehte die Kammern um und stellte sie in thermostatisierte Wannen, in denen die Temperatur und Luftfeuchte über der Kutikula durch einen leichten Luftstrom auf die Kutikula mit dem Spritzbelag einstellbar war (20°C, 60 % rh). In regelmäßigen Abständen wurden von einem Autosampler Aliquots entnommen und der Wirkstoffgehalt mit HPLC bestimmt.

Die Versuchsergebnisse gehen aus der folgenden Tabelle hervor. Bei den angegebenen Zahlen handelt es sich um Durchschnittswerte aus 8 bis 10 Messungen.

**Tabelle A**

| | | |
|---|---|---|
| Wirkstoff | Penetration nach 24h / % | |
| | SC + RME (1 g/l) + AS (1 g/l) | SL + RME (1 g/l) + AS (1 g/l) |
| Beispiel A.5 | 3 | 7 |
| RME = Rapsölmethylester (Einsatz formuliert als 500 EW, Konzentrationsangabe in g Wirkstoff /1)<br>AS = Ammoniumsulfat | | |

### Beispiel B

**Myzus persicae -Test (MYZUPE translaminar)**

Zur Herstellung einer zweckmäßigen Präparatelösung verdünnt man die Formulierung mit Wasser auf die gewünschte Konzentration. Bei erforderlicher Zugabe von Ammoniumsalzen und Penetrationsförderer wird die entsprechende Menge nach dem Verdünnen jeweils der fertigen Präparatelösung zupipettiert.

Kohlpflanzen (*Brassica oleracea*), die stark von der Grünen Pfirsichblattlaus (*Myzus persicae*) befallen sind,

werden durch Sprühen der Blattoberseite mit der Präparatelösung in der gewünschten Konzentration behandelt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigten z. B. die folgenden Verbindungen der Herstellungsbeispiele gute Wirksamkeit im Vergleich zu einer herkömmlichen Formulierung:

| Formulierung | Konzentration in g ai/ha | Abtötung in % nach 7[d] |
|---|---|---|
| **A.5 SL 050**<br>+ RME EW 500 + AMS (1 g ai/1) erfindungsgemäß | 3 | 90 |
| **A.5 SC 050**<br>+ RME EW 500 + AMS (1 g ai/1) Stand der Technik | 3 | 40 |
| AMS = Ammoniumsulfat | | |

## Beispiel C

Ca. 15 cm große Baumwollpflanzen der Sorte "Delta Opal" werden in ca. 8 $m^2$ großen Parzellen in drei Replikationen gegen Aphis gossypii unter Verwendung einer druckluftbetriebenen Rückenspritze (3 bar) behandelt. Dabei wird der Wirkstoff A.5 als SC050 und SL050 in der angebenen Aufwandmenge in einem Tankmix aus 1 g/l Ammoniumsulfat und 1 g/l a.i. Rapsölmethylester EW 500 mit einer Wasseraufwandmenge von 300 1/ha appliziert. Die Auswertung erfolgt 1 und 3 Tage nach der Appliktion indem man die Abtötung der Nymphen auf den Blättern bonitiert.

| Wirkstoff | Aufwandmenge | Wirkung Abbott (%) Aphis gossypii | |
|---|---|---|---|
| **A.5** | **g a.i. / ha** | **1d** | **3d** |
| SC 050 | 24 | 61.1 | 85.7 |
| SL 050 | 24 | 62.8 | 93.6 |

## Beispiel D

### Phaedon-Test (Spritzbehandlung)

| | |
|---|---|
| Lösungsmittel: | 78,0 Gewichtsteile Aceton<br>1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 500 g/ha:

Bsp. Nr. A1, A2, A3, A4, A6, A7, A8

**Beispiel E**

**Myzus-Test (MYZUPE Spritzbehandlung)**

| | |
|---|---|
| Lösungsmittel: | 78,0 Gewichtsteile Aceton<br>1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.
Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.
Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z. B. die folgenden Verbindungen der Herstellungsbeispiele eine Wirkung von ≥ 80 % bei einer Aufwandmenge von 500 g/ha:

Bsp. Nr. A1, A2, A3, A4, A5, A6, A7, A8

**Beispiel F**

**Tetranychus-Test, OP-resistent (TETRUR Spritzbehandlung)**

| | |
|---|---|
| Lösungsmittel: | 78,0 Gewichtsteile Aceton<br>1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.
Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.
Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.
Bei diesem Test zeigen z. B. die folgenden Verbindungen der Herstellungsbeispiele eine Wirkung von ≥ 80 % bei einer Aufwandmenge von 100 g/ha:

Bsp. Nr. A2, A5, A6

**Patentansprüche**

1. Verbindungen der Formel (I)

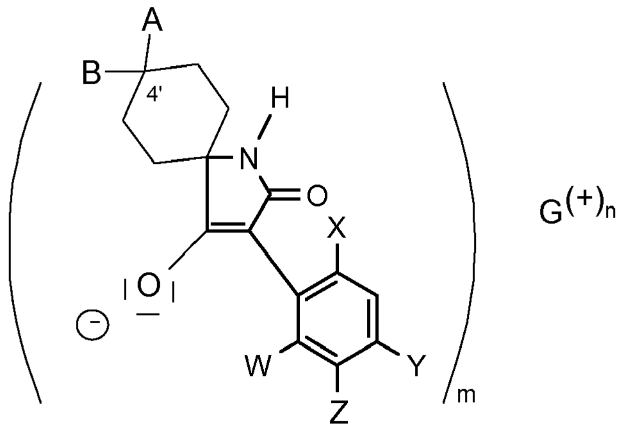

(I)

in welcher

W für Wasserstoff, Alkyl, Alkenyl, Alkinyl, Halogen, Alkoxy, Halogenalkyl, Halogenalkoxy oder Cyano steht,
X für Halogen, Alkyl, Alkenyl, Alkinyl, Alkoxy, Halogenalkyl, Halogenalkoxy, Nitro oder Cyano steht,

Y und Z unabhängig voneinander für Wasserstoff, Alkyl, Alkenyl, Alkinyl, Alkoxy, Halogen, Halogenalkyl, Halogenalkoxy, Cyano oder Nitro steht,
A und B und das Kohlenstoffatom, an das sie gebunden sind, für jeweils gegebenenfalls durch Alkyl, Halogenalkyl, Alkoxy, Alkoxyalkyl oder gegebenenfalls substituiertes Phenyl substituiertes fünf- bis siebengliedriges Ketal steht,

G für ein Metallionenäquivalent oder Ammoniumion steht,
m für die Zahl 1 oder 2 steht,
n für die Zahl 1 oder 2 steht.

Europäisches Patentamt

# EUROPÄISCHER RECHERCHENBERICHT

Nummer der Anmeldung
EP 08 15 3002

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2005 008021 A1 (BAYER CROPSCIENCE AG [DE]) 24. August 2006 (2006-08-24)<br>* Seite 57, Absatz 150 *<br>* Seite 18, Zeile 2 *<br>* Seite 100; Beispiel O *<br>* Seite 100; Beispiel q *<br>* Seiten 106-107; Anspruch 1 *<br>----- | 1 | INV.<br>C07D491/113<br>A01N43/90 |
| A | WO 2007/068427 A (BAYER CROPSCIENCE AG [DE]; FISCHER REINER [DE]; LEHR STEFAN [DE]; FEUC) 21. Juni 2007 (2007-06-21)<br>* Seite 89; Beispiel 1; Tabelle 1 *<br>* Seite 101, Zeilen 3,25; Anspruch 1 *<br>----- | 1 | |

RECHERCHIERTE SACHGEBIETE (IPC)

C07D
A01N

Der vorliegende Recherchenbericht wurde für alle Patentansprüche erstellt

2

| Recherchenort | Abschlußdatum der Recherche | Prüfer |
|---|---|---|
| München | 26. Juni 2008 | Gutke, Hans-Jürgen |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 08 15 3002

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-06-2008

| Im Recherchenbericht angeführtes Patentdokument | Datum der Veröffentlichung | Mitglied(er) der Patentfamilie | Datum der Veröffentlichung |
|---|---|---|---|
| DE 102005008021 A1 | 24-08-2006 | AR 053815 A1 | 23-05-2007 |
| | | AU 2006218154 A1 | 31-08-2006 |
| | | CA 2597777 A1 | 31-08-2006 |
| | | CN 101160049 A | 09-04-2008 |
| | | EP 1855529 A2 | 21-11-2007 |
| | | WO 2006089633 A2 | 31-08-2006 |
| | | KR 20070106554 A | 01-11-2007 |
| WO 2007068427 A | 21-06-2007 | AR 057994 A1 | 09-01-2008 |
| | | DE 102005059471 A1 | 12-07-2007 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0262399 A
- GB 2266888 A
- EP 355599 A
- EP 415211 A
- JP 12053670 A
- EP 377893 A
- EP 442077 A
- EP 442073 A
- EP 456063 A
- EP 521334 A
- EP 596298 A
- EP 613884 A
- EP 613885 A
- WO 9401997 A
- WO 9526954 A
- WO 9520572 A
- EP 0668267 A
- WO 9625395 A
- WO 9635664 A
- WO 9701535 A
- WO 9702243 A
- WO 9736868 A
- WO 9743275 A
- WO 9805638 A
- WO 9806721 A
- WO 9825928 A
- WO 9924437 A
- WO 9943649 A
- WO 9948869 A
- WO 9955673 A
- WO 0117972 A
- WO 0123354 A
- WO 0174770 A
- WO 03013249 A
- WO 03062244 A
- WO 2004007448 A
- WO 2004024688 A
- WO 04065366 A
- WO 04080962 A
- WO 04111042 A
- WO 05044791 A
- WO 05044796 A
- WO 05048710 A
- WO 05049596 A
- WO 05066125 A
- WO 05092897 A
- WO 06000355 A
- WO 06029799 A
- WO 06056281 A
- WO 06056282 A
- WO 06089633 A
- WO 07048545 A
- WO 07073856 A
- WO 07096058 A
- WO 07121868 A
- WO 07140881 A
- WO 9916748 A
- JP 14205984 A
- WO 06024411 A
- EP 86750 A
- EP 94349 A
- EP 191736 A
- EP 492366 A
- EP 174562 A
- EP 346620 A
- WO 9507897 A
- WO 9107874 A
- EP 269806 A
- EP 333131 A
- WO 9108202 A
- EP 582198 A
- EP 613618 A
- DE 2218097 A
- DE 2350547 A
- US 6235680 A
- WO 9966795 A
- US 6251827 A
- WO 95017817 A
- EP 0453086 A
- EP 0664081 A
- FR 2600494 A
- US 4844734 A
- US 5462912 A
- US 5538937 A
- US 030224939 A
- US 050009880 A
- US 050096386 A
- US 6645914 B
- EP 0036106 A2
- WO 9216108 A
- WO 07068427 A
- WO 07068428 A
- WO 9835553 A
- WO 0035278 A
- EP 0681865 A
- WO 9429268 A

**In der Beschreibung aufgeführte Nicht-Patentliteratur**


- **S. Suzuki et al.** *Chem. Pharm. Bull.,* 1967, vol. 15, 1120
- **von R. Schmierer ; H. Mildenberger.** *Liebigs Ann. Chem.,* 1985, 1095
- **Ito M.** *Bioscience, Biotechnology and Biochemistry,* 2003, vol. 67, 1230-1238
- **Baur et al.** *Pesticide Science,* 1967, vol. 51, 131-152